# EUROPEAN PATENT APPLICATION

(11) **EP 4 374 854 A1**
(43) Date of publication of application: **29.05.2024**
(21) Application number: 23166792.4
(22) Date of filing: 05.04.2023
(51) Int. Cl.: A61K 9/20, A61K 9/28

(54) **PHARMACEUTICAL MIXTURE COMPRISING AMORPHOUS PITOLISANT**

(30) Priority: 28.11.2022 IN 202211068391
(71) Applicant: Alfred E. Tiefenbacher (GmbH & Co. KG), 22767 Hamburg (DE)
(72) Inventor: PRATHAP, Vamshi Ramana, 502 319 Sangareddy (IN); MEYER, Claudia, 22767 Hamburg (DE); CHALAMALASETTI, Siva Ramanjaneyulu, 502 319 Sangareddy (IN); STEIERT, Nico, 22767 Hamburg (DE); STAVER, Ruslan, 22767 Hamburg (DE); JOSHI, Abhay Ramakant, 502 319 Sangareddy (IN); BOGOLU, Sindhu, 502 319 Sangareddy (IN)
(74) Representative: Ter Meer Steinmeister & Partner

(57) **Abstract**

The present invention relates to a pharmaceutical mixture comprising amorphous pitolisant free base or a pharmaceutically acceptable salt thereof and at least one pharmaceutically acceptable excipient. The present invention also related to an oral dosage form containing the pharmaceutical mixture and a method for treating narcolepsy with or without cataplexy.

## Description

### Background of the invention

The present invention relates to a pharmaceutical mixture comprising pitolisant free base or a pharmaceutically acceptable salt thereof, wherein pitolisant free base or a pharmaceutically acceptable salt thereof is present in the mixture in amorphous form, and at least one pharmaceutically acceptable excipient.

Further, the invention relates to a process for producing said pharmaceutical mixture comprising pitolisant and at least one pharmaceutically acceptable excipient and to the corresponding process of producing an oral dosage form containing the premix of the invention. Further, the invention relates to a method for treating narcolepsy with or without cataplexy.

### Technical Background

Pitolisant is a potent, orally active histamine H3-receptor antagonist/inverse agonist which, via its blockade of histamine auto-receptors enhances the activity of brain histaminergic neurons, a major arousal system with widespread projections to the whole brain. Pitolisant also modulates various neurotransmitter systems, increasing acetylcholine, noradrenaline and dopamine release in the brain. However, no increase in dopamine release in the striatal complex including nucleus accumbens was evidenced for pitolisant.

In the context of this invention, the term "pitolisant" refers to 1-[3-[3-(4-chlorophenyl)propoxy]propyl]-piperidine according to Formula (1).

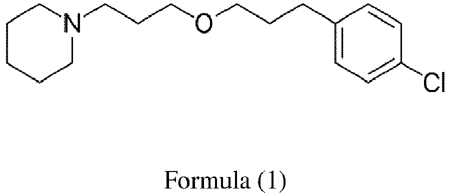

Pitolisant free base is reported to be an oil. Pitolisant is marketed under the tradename Wakix^{®} as film-coated tablets containing 5 mg and 20 mg crystalline pitolisant hydrochloride, corresponding to 4.45 mg and 17.8 mg pitolisant free base. Wakix^{®} are immediate release tablets to be administered once daily, in the morning.

It is commonly known that the dissolution behaviour of a drug depends on its solid state. Different crystalline forms of a drug usually exhibit different dissolution profiles, whereby amorphous forms are generally much more soluble than their crystalline counterparts. In addition, the chemical and physical stability of a drug are dependent on the solid state. Quite often, metastable crystalline or amorphous forms of a drug have to be stabilized in the pharmaceutical composition in order to prevent chemical degradation and interconversion of the crystalline forms/recrystallization of the amorphous form and, thus, fluctuations in the bioavailability.

EP 0 982 300 A2 discloses alkylamines as histamine H3 - receptor ligands. Example 78 discloses pitolisant as crystalline oxalate.

WO2006084833A1 is directed to crystalline pitolisant hydrochloride. The crystalline pitolisant hydrochloride as disclosed therein has an X-ray powder diffraction pattern with characteristic peaks at 11.2°, 19.9°, 20.7° and 34.1° (±0.2°2θ). The aqueous solubility of crystalline pitolisant hydrochloride (4 g/ml at 23°C) is higher than the aqueous solubility of the oxalate salt (0.025 g/ml at 23°C) disclosed in EP 0 982 300 A2.

In WO2006084833A1, solubility of pitolisant hydrochloride, hydrogen oxalate, hydrogen succinate and hydrogen maleate salts was carried out under different conditions: water at room temperature, pH 4.5 and pH 7.4. Results are shown in Table 1 below.

**Table 1: Solubility data**

| Salt | Solubility (% w/v) | | |
|---|---|---|---|
| | Water room T | pH 4.5 | pH 7.4 |
| Monohydrochloride | >100 | >100 | >100 |
| Hydrogen oxalate | 1-3 | 1-3 | 3-10 |
| Hydrogen succinate | >100 | >100 | >100 |
| Hydrogen maleate | >50 | >50 | 10-50 |

Also the melting points were determined. See Table 2 below.

**Table 2: Melting points**

| Conditions | Monohydrochloride | Hydrogen oxalate | Hydrogen succinate | Hydrogen maleate |
|---|---|---|---|---|
| N₂ atmosphere | 117.68 °C | 149.16 °C | 63.21 °C | 90.85 °C |
| O₂ atmosphere | 117.54 °C | 149.28 °C | 63.46 °C | 90.74 °C |

The four salts kept substantially unchanged their melting points independently of the oxidation conditions, therefore all of them can be regarded as equally stable.

In view of the above described state of the art, the objective underlying the present invention was the provision of a pharmaceutical mixture, in which pitolisant is physically and chemically stable and which shows a high solubility and bioavailability. This objective is attained by the subject matter as defined in the claims.

### Summary of the Invention

According to the present invention, the above-mentioned technical problems can be solved by a pharmaceutical mixture comprising
a) pitolisant free base or a pharmaceutically acceptable salt thereof, wherein pitolisant free base or a pharmaceutically acceptable salt thereof is present in the mixture in amorphous form, and
b) at least one pharmaceutically acceptable excipient.

In the pharmaceutical mixture of the present invention, pitolisant free base or a pharmaceutically acceptable salt thereof in amorphous form is stabilized by the at least one pharmaceutically acceptable excipient.

Surprisingly, it has been found that pitolisant free base or a pharmaceutically acceptable salt thereof in amorphous form is physically and chemically stable in the pharmaceutical mixture. In addition, pitolisant free base or a pharmaceutically acceptable salt thereof in amorphous form shows a high solubility and bioavailability. Moreover, pitolisant free base or a pharmaceutically acceptable salt thereof in amorphous form can be easily formulated in oral dosage forms.

According to one embodiment of the invention, the pharmaceutical mixture is a solid dispersion, wherein pitolisant free base or a pharmaceutically acceptable salt thereof is dispersed in a matrix containing the at least one pharmaceutically acceptable excipient, and wherein the solid dispersion is prepared by hot-melt extrusion.

According to a further embodiment of the invention, the pharmaceutical mixture is a solid dispersion, wherein pitolisant free base or a pharmaceutically acceptable salt thereof is dispersed in a matrix containing the at least one pharmaceutically acceptable excipient, and wherein the solid dispersion is prepared by spray granulation.

According to yet a further embodiment of the invention, the pharmaceutical mixture is a solid premix.

A further subject of the present invention is an oral dosage form comprising the pharmaceutical mixture of the invention and optionally further pharmaceutical excipient(s).

### Detailed Description of the Invention

The present invention concerns a pharmaceutical mixture comprising
a) pitolisant free base or a pharmaceutically acceptable salt thereof, wherein pitolisant free base or a pharmaceutically acceptable salt thereof is present in the mixture in amorphous form, and
b) at least one pharmaceutically acceptable excipient.

In a preferred embodiment the pharmaceutical mixture of the present invention comprises pitolisant free base or a pharmaceutically acceptable salt thereof as the sole pharmaceutically active agent.

In an alternative embodiment the pharmaceutical mixture of the present invention can comprise pitolisant in combination with further pharmaceutically active agent(s).

In one embodiment, pitolisant is present as free base. In a further embodiment, pitolisant is present as a pharmaceutically acceptable salt. Pharmaceutically acceptable salts of pitolisant include hydrochloride, hydrobromide, hydrojodide, sulfate, bisulfate, tatrate, nitrate, citrate, bitratrate, phosphate, malate, maleate, fumarate, succinate, oxalate.

In a preferred embodiment of the invention, the pharmaceutical mixture comprises a pharmaceutically acceptable salt of pitolisant, preferably pitolisant hydrochloride.

The term "pitolisant free base or a pharmaceutically acceptable salt thereof' also refers to pharmaceutically acceptable solvates, hydrates and mixtures thereof.

In the pharmaceutical mixture of the present invention, pitolisant free base or a pharmaceutically acceptable salt thereof is present in amorphous form.

The term "amorphous" is used in the context of this invention to designate the state of solid substances in which the components (atoms, ions or molecules, i.e. in the case of an amorphous salt of pitolisant the corresponding molecules) do not exhibit any periodic arrangement over a great range (=long-range order). In amorphous substances, the components are usually not arranged in a totally disordered fashion and completely randomly, but are rather distributed in such a way that a certain regularity and similarity to the crystalline state can be observed with regard to the distance from and orientation towards their closest neighbours (=short-range order). Amorphous substances consequently preferably possess a short-range order, but no long-range order. In contrast to anisotropic crystals, solid amorphous substances are isotropic. The amorphous substances can be distinguished experimentally from crystalline substances by means of X-ray diffraction, where the amorphous substances do not reveal clearly defined interferences, but normally only a few diffuse interferences with small diffraction angles.

In the context of this invention, the expression "pitolisant free base in amorphous form" preferably refers to a substance which consists of amorphous pitolisant. Alternatively, "pitolisant free base" may also contain small amounts of crystalline pitolisant, provided that no defined interferences in X-ray diffraction can be detected. A mixture containing 85 to 99.99% by weight amorphous pitolisant free base and 0.01 to 15% crystalline pitolisant free base is preferred, more preferably 95 to 99.9% by weight amorphous pitolisant free base and 0.1 to 5% crystalline pitolisant free base. In a particularly preferred embodiment amorphous pitolisant free base does not contain crystalline pitolisant free base at all. The crystalline proportion is determined by means of quantitative X-ray diffractometry according to the method of Hermans and Weidinger, wherein porcelain powder can be added as reference.

In the context of this invention, the expression "pharmaceutically acceptable salt of pitolisant in amorphous form" preferably refers to a substance which consists of amorphous pitolisant salt, e.g. hydrochloride. Alternatively, "pharmaceutically acceptable salt of pitolisant" may also contain small amounts of crystalline pitolisant salt, e.g. hydrochloride, components, provided that no defined interferences in X-ray diffraction can be detected. A mixture containing 85 to 99.99% by weight amorphous pitolisant salt and 0.01 to 15% crystalline pitolisant salt is preferred, more preferably 95 to 99.9% by weight amorphous pitolisant salt and 0.1 to 5% crystalline pitolisant salt. In a particularly preferred embodiment amorphous pitolisant salt does not contain crystalline pitolisant salt at all. The crystalline proportion is determined by means of quantitative X-ray diffractometry according to the method of Hermans and Weidinger, wherein porcelain powder can be added as reference.

In case the weight of pitolisant is mentioned in the present invention, this refers to the weight of pitolisant in form of the free base. Thus, for example, 20 mg of pitolisant hydrochloride corresponds to 17.8 mg pitolisant in form of the free base.

The pharmaceutical mixture of the present invention further comprises at least one pharmaceutically acceptable excipient. With the at least one pharmaceutically acceptable excipient it can be particularly ensured that the complete amount of pitolisant free base or a pharmaceutically acceptable salt thereof remains in an amorphous state and does convert to a crystalline substance.

In one embodiment, the at least one pharmaceutically acceptable excipient is selected from the group comprising gum arabic, sodium alginate, propylene glycol alginate, agar, gelatin, tragacanth, xanthan gum, polyvinylpyrrolidone, poly(vinylpyrrolidone/vinylacetate), polyvinylcaprolactam/polyvinylacetate/polyethylene glycol graft copolymer, polyethylene glycol/polyvinyl alcohol graft copolymer, polyvinyl alcohol, polyethylene oxide, polyethylene oxide/propylene oxide), macrogolglycerol hydroxystearate, D-atocopheryl polyethylene glycol succinate, poly(butyl methacrylate/2-dimethylaminoethyl methacrylate/methyl methacrylate), poly(ethyl acrylate/methyl methacrylate), poly(ethyl acrylate/methyl methacrylate/trimethylammonioethyl methacrylate chloride), polyethylene glycol, methyl cellulose, hydroxypropyl methylcellulose, hydroxypropyl cellulose, hydroxyethyl cellulose, carboxymethyl ethyl cellulose, sodium carboxymethyl cellulose, starch, pregelatinized starch, sodium starch glycolate, colloidal silicon dioxide, carboxymethylcellulose, polyvinyl acetate, gelatins, hypromellose phthalate, polyols and mixtures thereof.

Polyols are compounds, polymeric or not, with more than one hydroxyl group. Polyols include polyether polyols, polyester polyols, polycarbonate polyols and acrylic polyols as well as sugar alcohols. Polyether polyols may be further subdivided and classified as polyethylene oxide or polyethylene glycol (PEG), polypropylene glycol (PPG) and polytetrahydrofuran. Examples of sugar alcohols are mannitol, maltitol, sorbitol, xylitol, erythritol and isomalt. Polyols also include cyclodextrins and maltodextrins. Starch derivatives and cellulose derivatives are not polyols according to the present invention.

Preferably, the cyclodextrin is a β-cyclodextrin or derivative thereof. In an embodiment of the invention, the β-cyclodextrin or derivative thereof is hydroxypropyl β-cyclodextrin (HP-β-CD), sulfobutyl β-cyclodextrin (SBE-β-CD), methylated β-cyclodextrin, carboxymethyl-β-cyclodextrin or a mixture thereof, preferably hydroxypropyl β-5 cyclodextrin (HP-β-CD), sulfobutyl β-cyclodextrin (SBE-β-CD) or a mixture thereof.

In one embodiment, the at least one pharmaceutically acceptable excipient is a polymer.

In a preferred embodiment, the at least one pharmaceutically acceptable excipient is selected from mannitol, microcrystalline cellulose, hydroxypropyl cellulose, hydroxypropyl β-cyclodextrin, maltodextrin, copovidone, and mixtures thereof, more preferably from microcrystalline cellulose, hydroxypropyl β-cyclodextrin, maltodextrin, copovidone, and mixtures thereof.

In one preferred embodiment of the invention, the at least one pharmaceutically acceptable excipient further comprises mesoporous inorganic hygroscopic-stability increasing substances.

A mesoporous substance is a substance containing pores with diameters between 2 and 50 nm. Preferably, the mesoporous inorganic hygroscopic-stability increasing substances are selected from silica, magnesium aluminometasilicate and magnesium carbonate. Suitable mesoporous silica products are commercially available under the tradename Syloid^{®}. As an alternative to mesoporous silica, mesoporous magnesium aluminometasilicate may be used, e.g. the magnesium aluminometasilicates marketed under the tradename Neusilm^{®}. A further alternative is mesoporous magnesium carbonate, which is available under the tradename Upsalite^{®}.

In one embodiment, the pharmaceutical mixture of the invention can contain additional pharmaceutically acceptable excipients. The pharmaceutical excipients are excipients with which the person skilled in the art is familiar, such as those which are described in the European Pharmacopoeia (Ph.Eur.) and/or in the US Pharmacopoeia (USP).

In a preferred embodiment of the present invention the pharmaceutical mixture form can further comprise one or more excipients(s) selected from fillers (d), binders (e), disintegrants (f) and plasticizers (i).

Fillers (d) or diluents can be used to increase the bulk volume and weight of a low-dose drug to a limit at which a pharmaceutical dosage form can be formed. Fillers should fulfil several requirements, such as being chemically inert, non-hygroscopic, biocompatible, easily processable and possessing good biopharmaceutical properties. Examples of fillers are lactose (anhydrous or monohydrate), sucrose, glucose, mannitol, calcium hydrogen phosphate, calcium carbonate, (microcrystalline) cellulose and others.

Binders (e) may be added to the pharmaceutical formulation in order to ensure that oral dosage forms, preferably tablets, can be formed with the required mechanical strength. The binder can, for example, be starch, polyvinyl pyrrolidone or cellulose derivatives such as hydroxypropyl cellulose.

Disintegrants (f) are compounds which enhance the ability of the dosage form, preferably the ability of the tablet to break into smaller fragments when in contact with a liquid, preferably water. Preferred disintegrants are sodium carboxymethyl starch, cross-linked polyvinyl pyrrolidone (crospovidone), sodium carboxymethyl glycolate (for example Explotab^{®}), swelling polysaccharide, for example soy polysaccharide, carrageenan, agar, pectin, starch and derivatives thereof or protein, for example formaldehyde-casein, sodium bicarbonate or mixtures thereof. More preferred are sodium carboxymethyl cellulose and cross-linked polyvinyl pyrrolidone (crospovidone).

Plasticizers (i) are compounds which reduce the glass transition temperature of compounds and increase flexibility. Examples of plasticizer are a monomeric plasticizer, e.g. triethylcitrate, triacetin, dibutyl sebacate, diethyl phthalate, glycerylmonostearate, glycerine or propylene glycol, or a polymeric plasticizer such as polyethylene glycol or poly(ethylene oxide/propylene oxide).

In one embodiment, the pharmaceutical mixture contains pitolisant and the at least one pharmaceutically acceptable excipient in a weight ratio of 10:1 to 1:10, preferably of 5:1 to 1:5, and more preferably of 3:1 to 1:3 (total weight of pitolisant, calculated on the basis of the free base weight, to the total weight of the at least one pharmaceutically acceptable excipient).

It turned out that with the above ratio it can be particularly ensured that the complete amount of pitolisant free base or a pharmaceutically acceptable salt thereof remains in a non-crystalline state and does reconvert to a crystalline substance.

In one embodiment of the invention, the pharmaceutical mixture is a solid dispersion, wherein pitolisant free base or a pharmaceutically acceptable salt thereof is dispersed in a matrix containing the at least one pharmaceutically acceptable excipient, and wherein the solid dispersion is prepared by hot-melt extrusion.

In one embodiment, the at least one pharmaceutically acceptable excipient present in the solid dispersion prepared by hot melt extrusion is one of the excipients described above in view of the pharmaceutical mixture. Of these excipients, gum arabic, sodium alginate, propylene glycol alginate, agar, gelatin, tragacanth, xanthan gum are less preferred for a hot melt extrusion process, in particular when used as the only pharmaceutically acceptable excipient.

Pitolisant free base is an oily substance at room temperature, pitolisant hydrochloride has a melting point of about 118°C. The hot-melt extrusion used for the preparation of the solid dispersion of the present invention has to be conducted at temperatures below the degradation temperature of pitolisant free base or of the pharmaceutically acceptable salt thereof. Preferably, the hot-melt extrusion is conducted at a temperature of 40-180°C, more preferably at a temperature of 80170°C, most preferably 100-140°C depending on the salt of pitolisant used. The hot-melt extrusion has to be carried out at a temperature that allows the dissolution of the pitolisant free base or the pharmaceutically acceptable salt thereof used as starting material within the at least one pharmaceutically acceptable excipient. In principle, any crystalline form of pitolisant free base or pharmaceutically acceptable salt thereof as well as the amorphous form may be used for the preparation of the solid dispersion of the present invention.

As is commonly known by the skilled person, the melting temperature of the mixture comprising pitolisant free base or a pharmaceutically acceptable salt thereof and the at least one pharmaceutically acceptable excipient can be modified by the addition of a polymer or a plasticizer.

The temperature of the hot-melt extrusion may be decreased when using a pharmaceutically acceptable polymer, so that it is possible to process polymers with relatively high glass transitions temperatures. In addition, a pharmaceutically acceptable polymer, such as polyvinylpyrrolidone, poly(vinylpyrrolidone/vinylacetate) and polyvinylcaprolactam/polyvinylacetate/ polyethylene glycol graft copolymer, may serve as a solubility enhancer for pitolisant and may avoid (re)crystallization of the drug during dissolution in the intestines.

Typically, the solid dispersion prepared by hot-melt extrusion (i.e. the extrudates) contains pitolisant and the at least one pharmaceutically acceptable excipient in a weight ratio of 10:1 to 1:10, preferably of 5:1 to 1:5, and more preferably of 3:1 to 1:3 (total weight of pitolisant, calculated on the basis of the free base weight, to the total weight of the at least one pharmaceutically acceptable excipient).

In one further embodiment of the invention, the pharmaceutical mixture is a solid dispersion, wherein pitolisant free base or a pharmaceutically acceptable salt thereof is dispersed in a matrix containing the at least one pharmaceutically acceptable excipient, and wherein the solid dispersion is prepared by spray granulation.

In one embodiment, the at least one pharmaceutically acceptable excipient present in the solid dispersion prepared by spray granulation is one of the excipients described above in view of the pharmaceutical mixture.

In one preferred embodiment, the solid dispersion prepared by spray granulation is in form of granules. The spray granulation method is selected from top-spray, bottom spray and tangential spray. Preferably, the top-spray granulation method is used.

Typically, the solid dispersion prepared by spray granulation contains pitolisant and the at least one pharmaceutically acceptable excipient in a weight ratio of 10:1 to 1:10, preferably of 5:1 to 1:5, and more preferably of 3:1 to 1:3 (total weight of pitolisant, calculated on the basis of the free base weight, to the total weight of the at least one pharmaceutically acceptable excipient).

In one further embodiment of the invention, the pharmaceutical mixture is a solid premix.

The term "solid premix" is used herein to describe combinations of pitolisant free base or a pharmaceutically acceptable salt thereof and at least one pharmaceutically acceptable excipient, wherein individual particles of the components cannot be distinguished using techniques such as optical microscopy. In embodiments, the drug is considered as being uniformly or non-uniformly distributed over surfaces of excipient particles. In other embodiments, the premixes are considered to be in the nature of molecular dispersions, or solid solutions.

"Solid premix" as used herein further refers to mixtures of pitolisant free base or a pharmaceutically acceptable salt thereof and at least one pharmaceutically acceptable excipient, wherein pitolisant free base or a pharmaceutically acceptable salt thereof is dispersed within a matrix of the at least one pharmaceutically acceptable excipient. A solid premix as used herein is obtained by dissolving pitolisant free base or a pharmaceutically acceptable salt thereof and the at least one pharmaceutically acceptable excipient in a suitable solvent or mixture of solvents, followed by consequent evaporation of the solvent or mixture of solvents by spray drying, freeze-drying or evaporation in a rotary vacuum evaporator. Alternatively, the at least one pharmaceutically acceptable excipient is added to the solution of pitolisant free base or a pharmaceutically acceptable salt thereof in the suitable solvent or mixture of solvents to obtain a suspension, followed by consequent evaporation of the solvent or mixture of solvents by spray drying, freeze-drying or evaporation in a rotary vacuum evaporator.

Solid premixes as used herein exclude physical mixtures of pitolisant free base or a pharmaceutically acceptable salt thereof and the at least one pharmaceutically acceptable excipient, i.e. mixtures obtained by simply mixing pitolisant free base or a pharmaceutically acceptable salt thereof and the at least one pharmaceutically acceptable excipient. Further, solid premixes as used herein do not comprise solid dispersions obtained by hot-melt extrusion or spray granulation.

In one embodiment, the at least one pharmaceutically acceptable excipient present in the solid premix is one of the excipients described above in view of the pharmaceutical mixture.

Typically, the solid premix contains pitolisant and the at least one pharmaceutically acceptable excipient in a weight ratio of 10:1 to 1:10, preferably of 5:1 to 1:5, and more preferably of 3:1 to 1:3 (total weight of pitolisant, calculated on the basis of the free base weight, to the total weight of the at least one pharmaceutically acceptable excipient).

In a preferred embodiment the solid premix of the invention can have a particle size of 0.1 to 800 µm, preferably 1 to 300 µm, more preferably 2 to 200 µm, especially 5 to 100 µm. The particle size can be determined by means of the microscope "Leica DM2500P", wherein the premix was dispersed in Halocarbon oil 700. When determining the particle size by microscope the "longest dimension" of the particle is measured.

In one further embodiment of the invention, the pharmaceutical mixture of the present invention can be prepared by further processes, such as wet granulation by rapid mixer granulation, co-milling, hot melt granulation or co-micronization.

A rapid mixer granulator is a high-precision tool used for dry grinding and wet granulation of materials/ingredients in the same tub. Impeller and chopper of the rapid mixer granulator are responsible for mixing and break granules mass. The impeller mixes the dry powder uniformly and prepares a wet mass after the addition of the binder. Generally, the impeller works at a speed of 100-250 rpm after binder addition. The chopper helps break down a large mass of granules into tiny granules. A chopper may be used and operated at a speed of 1000-3000 rpm.

In one embodiment of the present invention, the formulation principle for stabilizing amorphous pitolisant free base or a pharmaceutically acceptable salt thereof is co-milling with at least one pharmaceutically acceptable excipient. In such embodiment, amorphous pitolisant free base or a pharmaceutically acceptable salt thereof can be dry co-milled or wet co-milled with the at least one pharmaceutically acceptable excipient. For dry co-milling the added excipient can be selected from polyvinylpyrrolidone, e.g. PVPK30, cellulose derivatives, such as, but not limited to, hydroxypropylcellulose (HPC), hydroxypropylmethylcellulose (HPMC), hydroxypropylmethylcellulose acetate succinate (HPMC-AS), hydroxypropylcellulose phthalate (HPMC-P), methylcellulose (MC), polyethyleneglycols, and earth alkali metal silicas and silicates, e.g. fumed silicas, precipitated silicas, calcium silicates, such as Zeopharm^{®} 600, or magnesium aluminometasilicates such as Neusilin US2.

Pharmaceutical mixtures obtained by dry co-milling preferably comprise between 10 to 50 %, more preferably 30 to 50 %, by weight of amorphous pitolisant free base or a pharmaceutically acceptable salt thereof.

A wet co-milled pitolisant (free base or a pharmaceutically acceptable salt thereof)-excipient composition is obtained by co-milling amorphous pitolisant free base or a pharmaceutically acceptable salt thereof with the other excipients in a suitable solvent, preferably medium chain fatty acid triglycerides such as those known and commercially available under the trade names Acomed^{®}, Myritol^{®}, Captex^{®}, Neobee M 5 F, Miglyol0812, Mazol^{®}, Sefsol^{®}860. Miglyol0812, a fractionated coconut oil, is especially the most preferred. The other excipients can be especially a polyethylene-/polypropylene-/polyethylene-oxide block copolymers, in particular Pluronic F68 and, optionally, a small amount of a surfactant, e.g. sodium dodecyl sulfate (SDS).

A wet co-milled pitolisant free base or a pharmaceutically acceptable salt thereof - excipient composition is obtained by co-milling amorphous pitolisant free base or a pharmaceutically acceptable salt thereof with the other excipients in a suitable solvent. Representative suitable solvents include, but are not limited to, pharmaceutically acceptable oils, preferably with an unsaturated component such as a vegetable oil; monoglycerides of medium chain fatty acids, such as Imwitor^{®} 308 or Capmul MCM C8; medium chain fatty acid triglycerides such as those known and commercially available under the trade names Acomed^{®}, Myritol^{®}, Captex^{®}, Neobee^{®}M 5 F, Miglyol0812, Mazol^{®}, Sefsol^{®}860; mixed mono-di-tri- glycerides such as Maisine^{®}; transesterified ethoxylated vegetable oils such as Labrafil^{®} M 2125 CS; glycerol triacetate; polyglycerol fatty acid esters such as Plurol Oleique CC497. The other excipients can be selected from cellulose derivatives such as hydroxypropylmethylcellulose, polyvinylpyrrolidone, polyethyleneglycols, polyethylene- /polypropylene-/polyethylene-oxide block copolymers such as Pluronic F68, polymethacrylates, sodium dodecyl sulfate, polyoxyethylene sorbitan fatty acid esters suchas Tween^{®} 80, bile salts such as sodium deoxycholate, polyoxyethylene mono esters of a saturated fatty acid such as Solutol^{®} HS 15, water soluble tocopheryl polyethylene glycol succinic acid esters such as Vitamin E TPGS. Especially, Pluronic F68 and optionally, a small amount of a surfactant, e.g. sodium dodecyl sulfate are examples of excipients that stabilize the amorphous form of pitolisant free base or a pharmaceutically acceptable salt thereof upon wet co-milling.

Melt granulation can be performed by agglomeration of a powder mixture of pitolisant and optional excipients with a molten binder as an at least one excipient. In one embodiment, the hot melt granulation process comprises blending pitolisant free base or a pharmaceutically acceptable salt thereof and at least one excipient, and heating the blend to a temperature of from the excipient melting point temperature to below the decomposition temperature of pitolisant free base or a pharmaceutically acceptable salt thereof. As the at least one excipient, the same as disclosed above for the pharmaceutical mixture can be used. The hot melt granulation process results in a melt granulation which comprises granules of the drug embedded in the at least one excipient. The heated blend is mixed until such melt granules are obtained. Preferably, the blend is heated to a temperature at which the pitolisant free base or a pharmaceutically acceptable salt thereof remains in solid form in the pitolisant-excipient mixture. A jacketed mixer or a hot melt extruder can be used to prepare a melt granulation.

The pharmaceutical mixture of the present invention can be applied in form of an oral dosage form, in particular in form of a solid oral dosage form. Thus, another object of the present invention is a solid oral dosage form comprising the pharmaceutical mixture according to the present invention.

In an embodiment, the oral dosage form contains 2 to 25 wt% pitolisant free base or a pharmaceutically acceptable salt thereof, calculated based on the free base, preferably 5 to 20 wt% pitolisant free base or a pharmaceutically acceptable salt thereof, more preferably 6 to 10 wt% pitolisant free base or a pharmaceutically acceptable salt thereof, calculated based on the free base, wherein the pitolisant free base or a pharmaceutically acceptable salt thereof is present in the oral dosage form in amorphous form.

In a further embodiment, the oral dosage form contains 8 to 75 wt% of at least one pharmaceutically acceptable excipient, wherein the pitolisant and the at least one pharmaceutically acceptable excipient are contained in the pharmaceutical mixture of the invention. That is, the at least one pharmaceutically acceptable excipient is one as defined above in combination with a pharmaceutical mixture of the present invention.

In one embodiment, the at least one pharmaceutically acceptable excipient comprises mesoporous inorganic hygroscopic-stability increasing substances. Typically, those substances can be contained in the oral dosage form in an amount of 0 to 30 wt.-%, preferably 10 to 20 wt.-%, based on the total weight of the oral dosage form.

In a preferred embodiment the present oral dosage form can preferably comprise further pharmaceutical excipient(s), in addition to those contained in the pharmaceutical mixture.

The pharmaceutical excipients are excipients with which the person skilled in the art is familiar, such as those which are described in the European Pharmacopoeia (Ph.Eur.) and/or in the US Pharmacopoeia (USP).

In a preferred embodiment of the present invention the oral dosage form can further comprise one or more excipients(s) selected from surfactants (c), fillers (d), binders (e), disintegrants (f), lubricants (g), glidants (h) and plasticizers (i).

Surfactants (c) can be regarded as substances lowering the interfacial tension between two phases, thus enabling or supporting the formation of dispersions or working as a solubilizer. Common surfactants are alkylsulfates (for example sodium lauryl sulfate), alkyltrimethylammonium salts, alcohol ethoxylates and the like. Surfactants can be typically used in an amount of 0 to 5 wt%, preferably of 0 to 2 wt%, based on the total weight of the oral dosage form.

Fillers (d) or diluents can be used to increase the bulk volume and weight of a low - dose drug to a limit at which a pharmaceutical dosage form can be formed. Fillers should fulfil several requirements, such as being chemically inert, non-hygroscopic, biocompatible, easily processable and possessing good biopharmaceutical properties. Examples of fillers are lactose, sucrose, glucose, mannitol, calcium carbonate, cellulose and others. Fillers can be typically used in an amount of 0 to 90 wt.-%, preferably 1 to 80%, more preferably 20 to 70%, based on the total weight of the dosage form.

Binders (e) may be added to the pharmaceutical formulation in order to ensure that oral dosage forms, preferably tablets, can be formed with the required mechanical strength. The binder can, for example, be starch, polyvinyl pyrrolidone (e.g. copovidone) or cellulose derivatives such as hydroxypropyl cellulose. Binders can be typically present in an amount of 0 to 60 wt%, preferably 20 to 50 wt%, based on the total weight of the pharmaceutical formulation.

Disintegrants (f) are compounds which enhance the ability of the dosage form, preferably the ability of the tablet to break into smaller fragments when in contact with a liquid, preferably water. Preferred disintegrants are sodium carboxymethyl starch, cross-linked polyvinyl pyrrolidone (crospovidone), sodium carboxymethyl glycolate (for example Explotab^{®}), swelling polysaccharide, for example soy polysaccharide, carrageenan, agar, pectin, starch and derivatives thereof or protein, for example formaldehyde-casein, sodium bicarbonate or mixtures thereof. More preferred are sodium carboxymethyl cellulose and cross-linked polyvinyl pyrrolidone (crospovidone). Disintegrants can be typically used in an amount of 0 to 20 wt%, preferably of 1 to 15 wt%, based on the total weight of the dosage form.

The function of lubricants (g) is reported to ensure that tablet formation and ejection can occur with low friction between the solid and the die wall. Further, lubricants can generally increase the powder flowability. The lubricant is preferably a stearate or fatty acid, more preferably an earth alkali metal stearate, such as magnesium stearate. The lubricant can be typically present in an amount of 0 to 5 wt%, preferably of about 0.1 to 3.0 wt%, based on the total weight of the dosage form.

Glidants (h) can also be used to improve the flowability. Traditionally, talc was used as glidant, but is nowadays nearly fully replaced by colloidal silica (for example Aerosil^{®}). Typically, the glidant can be present in an amount of 0 to 5 wt%, more preferably 0.1 to 3.0 wt%, based on the total weight of the dosage form.

Plasticizers (i) are compounds which reduce the glass transition temperature of compounds and increase flexibility. Examples of plasticizer are a monomeric plasticizer, e.g. triethylcitrate, triacetin, dibutyl sebacate, diethyl phthalate, glycerylmonostearate, glycerine or propylene glycol, or a polymeric plasticizer such as polyethylene glycol or poly(ethylene oxide/propylene oxide). Plasticizers can be typically used in an amount of 0 to 2 wt%, preferably of 0 to 1.5 wt%, based on the total weight of the oral dosage form. The plasticizer can be present in the core of the tablet and/or in the coating.

It lies in the nature of pharmaceutical excipients that they sometimes can perform more than one function in a pharmaceutical formulation. In this regard it is generally noted that due to the nature of pharmaceutical excipients it cannot be excluded that a certain compound meets the requirements of more than one of components (c) to (h). However, in order to enable an unambiguous distinction, it is preferred in the present application that one and the same pharmaceutical compound can only function as one of the compounds (c) to (h). For example, if microcrystalline cellulose functions as a filler (d), it cannot additionally function as a disintegrant (f), even though microcrystalline cellulose also exhibits a certain disintegrating effect.

The oral dosage form of the present invention can preferably comprise
2 to 25 wt% pitolisant free base or a pharmaceutically acceptable salt thereof, calculated based on the free base, preferably 5 to 20 wt% pitolisant free base or a pharmaceutically acceptable salt thereof, calculated based on the free base, wherein the pitolisant free base or a pharmaceutically acceptable salt thereof is present in the oral dosage form in amorphous form,
8 to 75 wt% of at least one pharmaceutically acceptable excipient, wherein the pitolisant and the at least one pharmaceutically acceptable excipient are contained in the pharmaceutical mixture of the invention,
0 to 5 wt% surfactant, preferably 0 to 2 wt% surfactant,
0 to 90 wt% filler, preferably 1 to 80 wt% filler,
0 to 60 wt% binder, preferably 20 to 50 wt% binder,
0 to 20 wt% disintegrant, preferably 1 to 15 wt% disintegrant,
0 to 5 wt% lubricant, preferably 0.1 to 3.0 wt% lubricant and
0 to 5 wt% glidant, preferably 0.1 to 3wt% glidant,
0 to 2 wt% plasticizer, preferably 0 to 1.5 wt% plasticizer,
based on the total weight of the oral dosage form.

The oral dosage form of the present invention can more preferably comprise
7 to 20 wt% pitolisant free base or a pharmaceutically acceptable salt thereof, calculated based on the free base, preferably 10 to 15 wt% pitolisant free base or a pharmaceutically acceptable salt thereof, calculated based on the free base, wherein the pitolisant free base or a pharmaceutically acceptable salt thereof is present in the oral dosage form in amorphous form,
0 to 90 wt% filler, preferably 1 to 80 wt% filler,
0 to 5 wt% surfactant, preferably 0 to 2 wt% surfactant,
0 to 60 wt% binder, preferably 20 to 50 wt% binder,
0 to 20 wt% disintegrant, preferably 1 to 15 wt% disintegrant,
0 to 3 wt% lubricant, preferably 0.1 to 3.0 wt% lubricant and
0 to 5 wt% glidant, preferably 0.1 to 3wt% glidant,
0 to 2 wt% plasticizer, preferably 0 to 1.5 wt% plasticizer,
based on the total weight of the oral dosage form.

The oral dosage form of the present invention can comprise
6 to 20 wt% pitolisant free base or a pharmaceutically acceptable salt thereof, calculated based on the free base, preferably 7 to 10 wt% pitolisant free base or a pharmaceutically acceptable salt thereof, calculated based on the free base, wherein the pitolisant free base or a pharmaceutically acceptable salt thereof is present in the oral dosage form in amorphous form,
10 to 40 wt% microcrystalline cellulose,
10 to 40 wt% mannitol,
4 to 8 wt% crospovidone,
1.5 to 4 wt% mesoporous silica,
10 to 30 wt% hydroxypropyl cellulose,
0.1 to 3 wt% talc, and
0.1 to 3 wt% magnesium stearate,
0 to 5wt% film coating,
based on the total weight of the oral dosage form.

The oral dosage form of the present invention can also comprise
6 to 20 wt% pitolisant free base or a pharmaceutically acceptable salt thereof, calculated based on the free base, preferably 7 to 10 wt% pitolisant free base or a pharmaceutically acceptable salt thereof, calculated based on the free base, wherein the pitolisant free base or a pharmaceutically acceptable salt thereof is present in the oral dosage form in amorphous form,
10 to 40 wt% microcrystalline cellulose,
10 to 40 wt% mannitol,
4 to 8 wt% crospovidone,
1.5 to 4 wt% mesoporous silica,
10 to 40 wt% hydroxypropyl β-cyclodextrin,
0.1 to 3 wt% talc, and
0.1 to 3 wt% magnesium stearate,
0 to 5wt% film coating,
based on the total weight of the oral dosage form.

The oral dosage form of the present invention can also comprise
6 to 20 wt% pitolisant free base or a pharmaceutically acceptable salt thereof, calculated based on the free base, preferably 7 to 10 wt% pitolisant free base or a pharmaceutically acceptable salt thereof, calculated based on the free base, wherein the pitolisant free base or a pharmaceutically acceptable salt thereof is present in the oral dosage form in amorphous form,
50 to 70 wt% microcrystalline cellulose,
4 to 8 wt% crospovidone,
1.5 to 4 wt% mesoporous silica,
10 to 25 wt% hydroxypropyl β-cyclodextrin,
0.1 to 3 wt% talc, and
0.1 to 3 wt% magnesium stearate,
0 to 5 wt% film coating,
based on the total weight of the oral dosage form.

The oral dosage form of the present invention can also comprise
6 to 20 wt% pitolisant free base or a pharmaceutically acceptable salt thereof, calculated based on the free base, preferably 7 to 10 wt% pitolisant free base or a pharmaceutically acceptable salt thereof, calculated based on the free base, wherein the pitolisant free base or a pharmaceutically acceptable salt thereof is present in the oral dosage form in amorphous form,
10 to 40 wt% microcrystalline cellulose,
4 to 6 wt% crospovidone,
10 to 20 wt% mesoporous silica,
20 to 40 wt% maltodextrin,
0.1 to 3 wt% talc, and
0.1 to 3 wt% magnesium stearate,
0 to 5wt% film coating,
based on the total weight of the oral dosage form.

The oral dosage form of the present invention preferably comprises:
8 to 12 wt% pitolisant free base or a pharmaceutically acceptable salt thereof, calculated based on the free base, wherein the pitolisant free base or a pharmaceutically acceptable salt thereof is present in the oral dosage form in amorphous form,
20 to 40 wt% maltodextrin,
30 to 50 wt% microcrystalline cellulose,
5 to 15 wt% crospovidone,
0.1 to 15 wt% mesoporous silica,
0.1 to 3 wt% talc, and
0.1 to 3 wt% magnesium stearate,
0 to 8 wt% film coating,
based on the total weight of the oral dosage form.

The oral dosage form of the present invention can also comprise
10 to 20 wt% pitolisant free base or a pharmaceutically acceptable salt thereof, calculated based on the free base, preferably 7 to 10 wt% pitolisant free base or a pharmaceutically acceptable salt thereof, calculated based on the free base, wherein the pitolisant free base or a pharmaceutically acceptable salt thereof is present in the oral dosage form in amorphous form,
20 to 40 wt% copovidone,
10 to 40 wt% microcrystalline cellulose,
4 to 8 wt% crospovidone,
0.1 to 3 wt% mesoporous silica,
0.1 to 3 wt% talc, and
0.1 to 3 wt% magnesium stearate,
0 to 7wt% film coating,
based on the total weight of the oral dosage form.

The oral dosage form of the present invention can also comprise
10 to 20 wt% pitolisant free base or a pharmaceutically acceptable salt thereof, calculated based on the free base, preferably 7 to 10 wt% pitolisant free base or a pharmaceutically acceptable salt thereof, calculated based on the free base, wherein the pitolisant free base or a pharmaceutically acceptable salt thereof is present in the oral dosage form in amorphous form,
30 to 50 wt% copovidone,
20 to 40 wt% microcrystalline cellulose,
5 to 15 wt% crospovidone,
0.1 to 3 wt% mesoporous silica,
0.1 to 3 wt% talc, and
0.1 to 3 wt% magnesium stearate,
0 to 7wt% film coating,
based on the total weight of the oral dosage form.

The oral dosage form of the present invention can also comprise
10 to 20 wt% pitolisant free base or a pharmaceutically acceptable salt thereof, calculated based on the free base, preferably 7 to 10 wt% pitolisant free base or a pharmaceutically acceptable salt thereof, calculated based on the free base, wherein the pitolisant free base or a pharmaceutically acceptable salt thereof is present in the oral dosage form in amorphous form,
25 to 45 wt% copovidone,
30 to 50 wt% microcrystalline cellulose,
2 to 7 wt% crospovidone,
0.1 to 3 wt% mesoporous silica,
0.1 to 3 wt% talc, and
0.1 to 3 wt% magnesium stearate,
0 to 7wt% film coating,
based on the total weight of the oral dosage form.

The oral dosage form of the present invention can also comprise
6 to 15 wt% pitolisant free base or a pharmaceutically acceptable salt thereof, calculated based on the free base, preferably 7 to 10 wt% pitolisant free base or a pharmaceutically acceptable salt thereof, calculated based on the free base, wherein the pitolisant free base or a pharmaceutically acceptable salt thereof is present in the oral dosage form in amorphous form,
25 to 45 wt% copovidone,
25 to 50 wt% microcrystalline cellulose,
5 to 10 wt% crospovidone,
0.1 to 3 wt% mesoporous silica,
0.1 to 3 wt% talc, and
0.1 to 3 wt% magnesium stearate,
0 to 7wt% film coating,
based on the total weight of the oral dosage form.

In a preferred embodiment of the invention the dosage form of the present invention can preferably comprise pitolisant free base or a pharmaceutically acceptable salt thereof in an amount from 4 to 30 mg, calculated based on the free base.

In a more preferred embodiment the dosage form of the present invention can preferably comprise pitolisant free base or a pharmaceutically acceptable salt thereof in an amount from 4 to 20 mg, calculated based on the free base. Especially preferred is an amount of pitolisant free base or a pharmaceutically acceptable salt thereof of 4.45 mg or 17.8 mg, calculated based on the free base.

In a still further embodiment of the present invention the oral dosage form can be a solid oral dosage form, preferably a capsule or a tablet, more preferably a tablet, in particular for peroral use. Alternatively, the solid oral dosage form can be filled as powder or granulate into devices like sachets or stick-packs.

In one embodiment, the oral dosage form can show modified release of the active ingredient. In that case the release profile of the oral dosage form, preferably of the tablet, according to USP method (apparatus 2; paddle, 1000 ml of 0.1 N HCl and 37°C, 75 rpm) after 2 hours indicates a content release of 0 to 90%, preferably of 10 to 80%, further preferably 15 to 75%, more preferably 20 to 50%.

In a more preferred embodiment of the invention the dosage form is for immediate release.

In one embodiment of immediate release, the content release of the dosage form after 5 minutes is 30% to 100%, preferably 50% to 95%, as determined by USP method (apparatus 2; paddle, 1000 ml of 0.1 N HCl and 37°C, 75 rpm).

In another embodiment of immediate release, the content release of the dosage form after 10 minutes is more than 70%, preferably more than 80%, as determined by USP method (apparatus 2; paddle, 1000 ml of 0.1 N HCl and 37°C, 75 rpm).

In another embodiment of immediate release, the content release of the dosage form after 15 minutes is 70 % to 100 %, as determined by USP method (apparatus 2; paddle, 1000 ml of 0.1 N HCl and 37°C, 75 rpm).

Further, the dosage form, preferably the tablet, of the invention preferably has a content uniformity, i.e. a content of active agent(s) which lies within the concentration of 90 to 110%, preferably 95 to 105% of the average content of the active agents). The "content uniformity" is determined with a test in accordance with Ph. Eur., 6.0, Chapter 2.9.6. According to that test, the content of the active agent of each individual tablet out of 20 tablets must lie between 90 and 110%, preferably between 95 and 105% of the average content of the active agent(s). Therefore, the content of the active drugs in each tablet of the invention differs from the average content of the active agent by at most 10%, preferably at most 5%.

In addition, the resulting tablet preferably has a friability of less than 5%, particularly preferably less than 2%, especially less than 1%. The friability is determined in accordance with Ph. Eur., 6.0, Chapter 2.9.7. The friability of tablets generally refers to tablets without coating.

The oral dosage form of the invention may be a peroral tablet which can be swallowed unchewed. The tablet can preferably be film-coated.

Generally, film coatings that do not affect the release of the active agent(s) and film coatings affecting the release of the active agent(s) can be employed with tablets according to invention. The film coatings that do not affect the release of the active agent(s) are preferred.

Preferred examples of film coatings which do not affect the release of the active ingredient can be those including poly(meth)acrylate, methylcellulose (MC), hydroxypropyl methylcellulose (HPMC), hydroxypropyl cellulose (HPC), hydroxyethyl cellulose (HEC), polyvinylpyrrolidone (PVP) and mixtures thereof. These polymers can have a weight-average molecular weight of 10,000 to 150,000 g/mol.

A further example of film coating which does not affect the release of the active ingredient is Opadry II^{®} 85F580019 white comprising polyvinyl alcohol, talc, macrogol/PEG, and titanium dioxide.

In an alternative preferred embodiment, the film coating can affect the release of the active agent. Examples for film coatings affecting the release of the active agent are gastric juice-resistant film coatings and retard coatings.

In a preferred embodiment the film can have a thickness of 2 µm to 150 µm, preferably from 10 to 100 µm, more preferably from 20 to 60 µm.

In one embodiment, the hardness of the uncoated tablet is 10-100 N, preferably 15-70 N, as determined as per USP <1217> or Ph. Eur. 2.9.8.

In one embodiment, the oral dosage form of the present invention contains amorphous pitolisant as defined above even after 6 months of storage at 40°C/75%RH in a HDPE bottle containing desiccant or in an alu-alu blister. That is, the XRPD of the tablet is immediately after production and after storage essentially the same. Preferably, no peaks characteristic of crystalline pitolisant are detectable after 6 months of storage at 40°C/75%RH in a HDPE bottle containing desiccant or in an alu-alu blister.

The present invention further relates to a method for producing a solid dispersion according to the invention, wherein pitolisant free base or a pharmaceutically acceptable salt thereof is dispersed in a matrix containing the at least one pharmaceutically acceptable excipient, and wherein the solid dispersion is prepared by hot-melt extrusion.

Hence, a further subject of the present invention is a method for preparing a solid dispersion comprising pitolisant free base or a pharmaceutically acceptable salt thereof, wherein pitolisant free base or a pharmaceutically acceptable salt thereof is present in the mixture in amorphous form, and at least one pharmaceutically acceptable excipient, comprising the steps of
(i) providing pitolisant free base or a pharmaceutically acceptable salt thereof, preferably in crystalline form, and at least one pharmaceutically acceptable excipient,
(ii) optionally blending the components from step (i),
(iii) hot-melt extruding the mixture of step (i) or the blend of step (ii) to obtain a solid dispersion in form of extrudates,
(iv) optionally milling the extrudates from step (iii) to form granulates, and
(v) optionally sieving the extrudate of step (iii) or the milled extrudates of step (iv).

Generally, the comments made above for pitolisant and the at least one pharmaceutically acceptable excipient can also apply to the method of the present invention.

In step (i) pitolisant free base or a pharmaceutically acceptable salt thereof and at least one pharmaceutically acceptable excipient are provided. Pitolisant free base or a pharmaceutically acceptable salt thereof can preferably be provided in crystalline form. Further, free base or a pharmaceutically acceptable salt thereof can be provided in micronized form. The step of micronization can preferably be carried out by milling, such as in an air jet mill.

In the optional step (ii), pitolisant free base or a pharmaceutically acceptable salt thereof and at least one pharmaceutically acceptable excipient can be blended in order to provide a composition having a homogenous distribution of pitolisant free base or a pharmaceutically acceptable salt thereof within the resulting blend comprising pitolisant free base or a pharmaceutically acceptable salt thereof and at least one pharmaceutically acceptable excipient. Blending can be carried out with conventional mixing devices, e.g. in a free-fall mixer like Turbula^{®} T10B (Bachofen AG, Switzerland). Blending can be carried out e.g. for 1 minute to 30 minutes, preferably for 2 minutes to less than 10 minutes. Preferably, step (ii) is carried out in order to ensure a homogenous blend before step (iii).

In step (iii), hot-melt extrusion is carried out in an extruder, e.g. Thermo Fischer twin screw extruder suitable for pharmaceutical purposes. Optionally, a degassing unit may be employed during hot-melt extrusion processing.

Pitolisant free base is an oily substance at room temperature, pitolisant hydrochloride has a melting point of about 118°C. The hot-melt extrusion has to be conducted at temperatures below the degradation temperature of pitolisant free base or of the pharmaceutically acceptable salt thereof. Preferably, the hot-melt extrusion is conducted at a temperature of 40-180°C, more preferably at a temperature of 80-170°C, most preferably at a temperature of 100-140°C, depending on the salt of pitolisant used. The hot-melt extrusion has to be carried out at a temperature that allows the dissolution of the pitolisant free base or the pharmaceutically acceptable salt thereof used as starting material within the at least one pharmaceutically acceptable excipient. In principle, any crystalline form of pitolisant free base or pharmaceutically acceptable salt thereof as well as the amorphous form may be used for the preparation of the solid dispersion of the present invention.

In step (iv) milling of the extrudates from step (iii) can preferably be performed in conventional milling apparatuses, such as in a ball mill, air jet mill, pin mill, classifier mill, cross beater mill, disk mill, mortar grinder or a rotor mill. A ball mill is preferably used.

The milling time is preferably 5 minutes to 6 hours, preferably 10 minutes to 4 hours, more preferably 15 minutes to 2 hours.

It is preferred that the optional step (v) of sieving the mixture of step (iii) or (iv) can be carried out with a sieve having a mesh size of 25 to 1000 µm, preferably 50 to 800 µm, especially 100 to 600 µm.

Further, the subject of the present invention relates to a method for preparing the oral dosage form of the invention comprising the steps:
(i) providing pitolisant free base or a pharmaceutically acceptable salt thereof, preferably in crystalline form, and at least one pharmaceutically acceptable excipient,
(ii) optionally blending the components from step (i),
(iii) hot-melt extruding the mixture of step (i) or the blend of step (ii) to obtain a solid dispersion in form of extrudates,
(iv) optionally milling the extrudates from step (iii) to form granulates,
(v) optionally sieving the extrudate of step (iii) or the milled extrudates of step (iv)
(vi) optionally adding further excipient(s) to the mixture of step (iii), step (iv) or step (v),
(vii) optionally granulating the mixture of step (iii) or step (vi) and
(viii) processing the mixture of step (iii) or step (vi) or the granulates of step (iv), step (v) or step (vii) into an oral dosage form.

In steps (i) to (v) a solid dispersion formed by hot-melt extrusion according to the present invention is provided, i.e. all the above process steps (i) to (v) leading to the present solid dispersion formed by hot-melt extrusion also apply to the process for preparing the present oral dosage form.

In step (vi) additional further excipient(s) and/or further pharmaceutically active agent can optionally be added to the mixture of step (iii), step (iv) or step (v). During or after the addition of the optional excipients and/or further pharmaceutically active agent the resulting mixture can preferably be blended. Blending can be carried out with the conventional mixing devices. The excipients can preferably be selected from the excipients (c) to (i) as described above.

In step (vii) the mixture of step (iii) or step (vi) can be optionally granulated.

"Granulating" is generally understood to mean the formation of relatively coarse or granular aggregate material as a powder by assembling and/or aggregating finer powder particles (agglomerate formation or build-up granulation) and/or the formation of finer granules by breaking up coarser aggregates (disintegration or break-down granulation).

Granulation can conventionally mean wet or dry granulation.

Dry granulation, which is preferred, is generally carried out by using pressure or temperature. In a preferred embodiment of the invention, granulating the mixture from step (iii) or step (vi) can be performed for example by "slugging" using a large heavy-duty rotary press and breaking up the slugs into granulates with a hammer mill or by roller compaction using for example roller compactors by Powtec or Alexanderwerk. The granulates are then optionally screened.

The mixture of step (iii) can be preferably granulated by milling the extrudates, as explained for optional step (iv).

In step (viii) the mixture of step (iii) or step (vi) or the granulates of step (iv), step (v) or step (vii) can be processed into a solid oral dosage form. Processing the mixture of step (iii) or step (vi) or the granulates of step (iv), step (v) or step (vii) into a solid oral dosage form can preferably comprise filling said mixture of step (iii) or step (vi) or the granulates of step (iv), step (v) or step (vii) into capsules, preferably hard gelatine capsules.

More preferred, processing the mixture of step (iii) or step (vi) or the granulates of step (iv), step (v) or step (vii) into tablets can be carried out by compressing said formulation on a rotary press, e.g. on a Fette^{®} (Fette GmbH, Germany) or a Riva^{®} piccola (Riva, Argentina). The main compression force can range from 1 to 50 kN, preferably from 3 to 40 kN. The resulting tablets can have a hardness of 30 to 400 N, more preferred of 50 to 250 N, particularly preferably of 30 to 180 N, more preferably 40 to 150 N, wherein the hardness can be measured according to Ph.Eur. 6.0, Chapter 2.9.8. For the optional filling of the formulation into capsules dependent dosing systems (for example an auger) or preferably independent dosing systems (for example MG2, Matic (IMA)) can be used.

The present invention further relates to a method for producing a solid dispersion according to the invention, wherein pitolisant free base or a pharmaceutically acceptable salt thereof is dispersed in a matrix containing the at least one pharmaceutically acceptable excipient, and wherein the solid dispersion is prepared by spray granulation.

Hence, a further subject of the present invention is a method for preparing a solid dispersion comprising pitolisant free base or a pharmaceutically acceptable salt thereof, wherein pitolisant free base or a pharmaceutically acceptable salt thereof is present in the mixture in amorphous form, and at least one pharmaceutically acceptable excipient, comprising the steps of
(i) providing pitolisant free base or a pharmaceutically acceptable salt thereof, preferably in crystalline form, and at least one pharmaceutically acceptable excipient,
(ii) dissolving the mixture of step (i) in a solvent,
(iii) providing a mixture of further pharmaceutically acceptable excipients,
(iv) spraying the solution of step (ii) on the mixture of step (iii) to form granules,
(v) optionally sieving the granules of step (iii).

Generally, the comments made above for pitolisant and the at least one pharmaceutically acceptable excipient can also apply to the method of the present invention.

In step (i) pitolisant free base or a pharmaceutically acceptable salt thereof and at least one pharmaceutically acceptable excipient are provided. Pitolisant free base or a pharmaceutically acceptable salt thereof can preferably be provided in crystalline form. Further, free base or a pharmaceutically acceptable salt thereof can be provided in micronized form. The step of micronization can preferably be carried out by milling, such as in an air jet mill. Optionally, pitolisant free base or a pharmaceutically acceptable salt thereof and at least one pharmaceutically acceptable excipient can be blended in order to provide a composition having a homogenous distribution of pitolisant free base or a pharmaceutically acceptable salt thereof within the resulting blend comprising pitolisant free base or a pharmaceutically acceptable salt thereof and at least one pharmaceutically acceptable excipient. Blending can be carried out with conventional mixing devices, e.g. in a free-fall mixer like Turbula^{®} T10B (Bachofen AG, Switzerland). Blending can be carried out e.g. for 1 minute to 30 minutes, preferably for 2 minutes to less than 10 minutes. In view of step (ii), blending is not preferred.

In step (ii), the mixture of step (i) is dissolved in a solvent. Typical solvents include water, ethanol and isopropanol either alone or in combination. Preferably, the solvent is water.

In step (iii), a mixture of further pharmaceutically acceptable excipients is provided. As excipients, those disclosed above in view of the pharmaceutical mixture can be used. Preferably, the further pharmaceutically acceptable excipients are selected from fillers (d), binders (e), disintegrants (f) and plasticizers (i). Typically, the further pharmaceutically acceptable excipients comprise a filler and optionally a further excipient, such as a binder (e), a disintegrant (f), a plasticizer (i) and/or a mesoporous inorganic hygroscopic-stability increasing substance as disclosed above.

In one preferred embodiment, the mesoporous inorganic hygroscopic-stability increasing substance as disclosed above, if present, is added in step (i), but not in step (iii).

Optionally, the further pharmaceutically acceptable excipients can be blended in order to provide a composition having a homogenous distribution. Blending can be carried out with conventional mixing devices, e.g. in a free-fall mixer like Turbula^{®} T10B (Bachofen AG, Switzerland). Blending can be carried out e.g. for 1 minute to 30 minutes, preferably for 2 minutes to less than 10 minutes. In view of step (ii), blending is not preferred.

In step (iv), the solution of step (ii) is sprayed on the mixture of step (iii) to obtain granules. The spray granulation method is selected from top-spray, bottom spray and tangential spray. Preferably, the top-spray granulation method is used. Spray granulation is generally carried out in a fluid bed reactor.

Exemplary process parameters for the top-spray granulation can be:

| **Inlet (°C)** | **Product (°C)** | **Exhaust (°C)** | **Atomization (bar)** | **Blower (%)** | **Airflow (CFM)** | **Spray (RPM)** | **RH** | |
|---|---|---|---|---|---|---|---|---|
| | | | | | | | **Inlet** | **Outlet** |
| 40-70, pref. 60 -65 | 25-60, pref. 30 - 45 | 25-60, pref. 29 - 40 | 0.5-1.5, pref. 0.8 - 1.0 | 10 to 100, pref. 17 -50 | 10 to 100, pref. 15 - 48 | 5 to 200, pref. 5 -30 | 0-10, pref., 0-5 | 5-60, pref. 40-58 |

It is preferred that the optional step (v) of sieving the granules of step (iv) can be carried out with a sieve having a mesh size of 25 to 1000 µm, preferably 50 to 800 µm, especially 100 to 600 µm.

Further, the subject of the present invention relates to a method for preparing the oral dosage form of the invention comprising the steps:
(i) providing pitolisant free base or a pharmaceutically acceptable salt thereof, preferably in crystalline form, and at least one pharmaceutically acceptable excipient,
(ii) dissolving the mixture of step (i) in a solvent,
(iii) providing a mixture of further pharmaceutically acceptable excipients,
(iv) spraying the solution of step (ii) on the mixture of step (iii) to form granules,
(v) optionally sieving the granules of step (iii).
(vi) optionally adding further excipient(s) to the mixture of step (iv) or step (v),
(vii) optionally granulating the mixture of step (vi), and
(viii) processing the granulates of step (iv) or step (v) or the granulates of step (vii) into an oral dosage form.

In steps (i) to (v) a solid dispersion formed by spray granulation according to the present invention is provided, i.e. all the above process steps (i) to (v) leading to the present solid dispersion formed by spray granulation also apply to the process for preparing the present oral dosage form.

In step (vi) additional further excipient(s) and/or further pharmaceutically active agent can optionally be added to the mixture of step (iv) or step (v). During or after the addition of the optional excipients and/or further pharmaceutically active agent the resulting mixture can preferably be blended. Blending can be carried out with the conventional mixing devices. The excipients can preferably be selected from the excipients (c) to (i) as described above.

In one preferred embodiment, the mesoporous inorganic hygroscopic-stability increasing substance as disclosed above, if present, is added in step (i) and/or step (vi), but not in step (iii).

In step (vii) the mixture of step (vi) can be optionally granulated.

"Granulating" is generally understood to mean the formation of relatively coarse or granular aggregate material as a powder by assembling and/or aggregating finer powder particles (agglomerate formation or build-up granulation) and/or the formation of finer granules by breaking up coarser aggregates (disintegration or break-down granulation).

Granulation can conventionally mean wet or dry granulation.

Dry granulation, which is preferred, is generally carried out by using pressure or temperature. In a preferred embodiment of the invention, granulating the mixture from step (vi) can be performed for example by "slugging" using a large heavy-duty rotary press and breaking up the slugs into granulates with a hammer mill or by roller compaction using for example roller compactors by Powtec or Alexanderwerk. The granulates are then optionally screened.

In step (viii) the granulates of step (iv) or step (v) or the granulates of step (vii) can be processed into a solid oral dosage form. Processing the granulates of step (iv) or step (v) or the granulates of step (vii) into a solid oral dosage form can preferably comprise filling said granulates of step (iv) or step (v) or the granulates of step (vii) into capsules, preferably hard gelatine capsules.

More preferred, processing the granulates of step (iv) or step (v) or the granulates of step (vii) into tablets can be carried out by compressing said formulation on a rotary press, e.g. on a Fette^{®} (Fette GmbH, Germany) or a Riva^{®} piccola (Riva, Argentina). The main compression force can range from 1 to 50 kN, preferably from 3 to 40 kN. The resulting tablets can have a hardness of 30 to 400 N, more preferred of 50 to 250 N, particularly preferably of 30 to 180 N, more preferably 40 to 150 N, wherein the hardness can be measured according to Ph.Eur. 6.0, Chapter 2.9.8. For the optional filling of the formulation into capsules dependent dosing systems (for example an auger) or preferably independent dosing systems (for example MG2, Matic (IMA)) can be used.

The present invention further relates to a method for producing a solid premix according to the invention. Hence, a further subject of the present invention is a method for preparing a solid premix comprising pitolisant free base or a pharmaceutically acceptable salt thereof, wherein pitolisant free base or a pharmaceutically acceptable salt thereof is present in the mixture in amorphous form, and at least one pharmaceutically acceptable excipient, comprising the steps of
(i) providing pitolisant free base or a pharmaceutically acceptable salt thereof, preferably in crystalline form, and at least one pharmaceutically acceptable excipient,
(ii) dissolving the components from step (i) in a solvent,
(iii) evaporating the solvent of step (iii) to obtain a solid premix, and
(iv) optionally milling and/or sieving the mixture from step (iii).

Generally, the comments made above for pitolisant and the at least one pharmaceutically acceptable excipient can also apply to the method of the present invention.

In step (i) pitolisant free base or a pharmaceutically acceptable salt thereof and at least one pharmaceutically acceptable excipient are provided. Pitolisant free base or a pharmaceutically acceptable salt thereof can preferably be provided in crystalline form. Further, free base or a pharmaceutically acceptable salt thereof can be provided in micronized form. The step of micronization can preferably be carried out by milling, such as in an air jet mill. Optionally, pitolisant free base or a pharmaceutically acceptable salt thereof and at least one pharmaceutically acceptable excipient can be blended in order to provide a composition having a homogenous distribution of pitolisant free base or a pharmaceutically acceptable salt thereof within the resulting blend comprising pitolisant free base or a pharmaceutically acceptable salt thereof and at least one pharmaceutically acceptable excipient. Blending can be carried out with conventional mixing devices, e.g. in a free-fall mixer like Turbula^{®} T10B (Bachofen AG, Switzerland). Blending can be carried out e.g. for 1 minute to 30 minutes, preferably for 2 minutes to less than 10 minutes.

In step (ii), the components of step (i) are dissolved in a solvent. The selection of the solvent is not limited, as long as the components of step (i) can be dissolved at 25°C within at most 30 min by stirring at 50 rpm. Preferred solvents include alcohols (e.g. methanol, ethanol, n-propanol , isopropanol, and butanol), ketones (e.g. acetone, methyl ethyl ketone and methyl isobutyl ketone), esters (e.g. ethyl acetate and propyl acetate) and various other solvents such as acetonitrile, methylene chloride, chloroform , hexane, toluene, tetrahydrofurane, cyclic ethers, and 1, 1 ,1-trichloroethane. Lower volatility solvents, such as dimethyl acetamide or dimethylsulfoxide can also be used. Mixtures of solvents, such as 20% ethanol and 80% acetone, can also be used, as can mixtures with water as long as the drug and if necessary the matrix agent are sufficiently soluble to make the process practicable.

Preferably, the solvent is selected from water, methanol, ethanol, acetone, ethylacetate, THF, diethyl ether, tert-butyl methyl ether, and mixtures thereof.

In step (iii), the solvent of step (iii) is evaporated to obtain a solid premix by spray drying, freeze-drying or evaporation in a rotary vacuum evaporator. After evaporation of the solvent, the obtained solid premix is optionally dried at low vacuum (50 to 200 mbar) at a temperature of 30°C to 50°C for 8 to 24 h.

In step (iv) optional milling of the mixture from step (iii) can preferably be performed in conventional milling apparatuses, such as in a ball mill, air jet mill, pin mill, classifier mill, cross beater mill, disk mill, mortar grinder or a rotor mill. A ball mill is preferably used.

The milling time is preferably 5 minutes to 6 hours, preferably 10 minutes to 4 hours, more preferably 15 minutes to 2 hours.

It is preferred that the optional step (iv) of sieving the mixture of step (iii) can be carried out with a sieve having a mesh size of 25 to 1000 µm, preferably 50 to 800 µm, especially 100 to 600 µm.

Further, the subject of the present invention relates to a method for preparing the oral dosage form of the invention comprising the steps:
(i) providing pitolisant free base or a pharmaceutically acceptable salt thereof, preferably in crystalline form, and at least one pharmaceutically acceptable excipient,
(ii) dissolving the components from step (i) in a solvent,
(iii) evaporating the solvent of step (iii) to obtain a solid premix, and
(iv) optionally milling and/or sieving the mixture from step (iii).
(v) optionally adding further excipient(s) to the mixture of step (iii) or step (iv),
(vi) optionally granulating the mixture of step (iii), step (iv) or step (v) and
(vii) processing the mixture of step (iii), step (iv), or step (v) or the granulates of step (vi) into an oral dosage form.

In steps (i) to (iv) a solid premix according to the present invention is provided, i.e. all the above process steps (i) to (iv) leading to the present solid premix also apply to the process for preparing the present oral dosage form.

In step (v) additional further excipient(s) and/or further pharmaceutically active agent can optionally be added to the mixture of step (iii) or step (iv). During or after the addition of the optional excipients and/or further pharmaceutically active agent the resulting mixture can preferably be blended. Blending can be carried out with the conventional mixing devices. The excipients can preferably be selected from the excipients (c) to (h) as described above.

In step (vi) the mixture of step (iii), step (iv) or step (v) can be optionally granulated.

"Granulating" is generally understood to mean the formation of relatively coarse or granular aggregate material as a powder by assembling and/or aggregating finer powder particles (agglomerate formation or build-up granulation) and/or the formation of finer granules by breaking up coarser aggregates (disintegration or break-down granulation).

Granulation can conventionally mean wet or dry granulation.

Dry granulation, which is preferred, is generally carried out by using pressure or temperature. In a preferred embodiment of the invention, granulating the mixture from step (iii), step (iv) or step (v) can be performed for example by "slugging" using a large heavy-duty rotary press and breaking up the slugs into granulates with a hammer mill or by roller compaction using for example roller compactors by Powtec or Alexanderwerk. The granulates are then optionally screened.

In step (vii) the mixture of step (iii), step (iv) or step (v) or the granulates of step (vi) can be processed into a solid oral dosage form. Processing the mixture of step (iii), step (iv) or step (v) or the granulates from step (vi) into a solid oral dosage form can preferably comprise filling said mixture of step (iii), step (iv) or step (v) or said granulates from step (vi) into capsules, preferably hard gelatine capsules.

More preferred, processing the mixture of step (iii), step (iv) or step (v) or the granulates from step (vi) into tablets can be carried out by compressing said formulation on a rotary press, e.g. on a Fette^{®} (Fette GmbH, Germany) or a Riva^{®} piccola (Riva, Argentina). The main compression force can range from 1 to 50 kN, preferably from 3 to 40 kN. The resulting tablets can have a hardness of 30 to 400 N, more preferred of 50 to 250 N, particularly preferably of 30 to 180 N, more preferably 40 to 150 N, wherein the hardness can be measured according to Ph.Eur. 6.0, Chapter 2.9.8. For the optional filling of the formulation into capsules dependent dosing systems (for example an auger) or preferably independent dosing systems (for example MG2, Matic (IMA)) can be used.

Further, the invention relates to a method for treating narcolepsy with or without cataplexy, comprising administering to a subject in need thereof a therapeutically effective amount of the pharmaceutical mixture of the invention or the dosage form of the invention.

### EXAMPLES

### Hot-melt Extrusion

Hot melt extrusion was performed with a Pharma 11 Twin-screw hot melt extruder from Thermo Fisher Scientific Inc. The used film coating system Opadry II^{®} 85F580019 white comprises polyvinyl alcohol, talc, macrogol/PEG, and titanium dioxide.

### Spray Granulation

For spray granulation, a GPCG 1.1 fluid bed granulator from Glatt GmbH (Binzen, Germany) was used.

### X-Ray Powder Diffraction

### XRD method

The XRD measurements were performed using X-ray source with Cu K-alpha radiation, Empyrean system (or equivalent), PIXcel detector, divergence slit 0.25° fixed, anti-scattering slit 0.5°, seller slits 2x0.02 radians, current 40mA, voltage 45kV, 2° - 40 ° 2θ, spinning 30RPM, step size 0.013° with total measurement time 1hr at room temperature.

### Sample preparation for XRD

1. Grind about two tablets to fine powder using Agate mortar and pestle gently.
   Prepare the sample (approx. 350 mg) using PANalytical sample preparation kit by 'Back loading technique'. The sample surface should be smooth and in parallel to sample holder surface. Clean the outer edges of the holder to avoid sample contaminations.
2. Place the prepared sample holder carefully on the sample stage of the XRD instrument and analyze as per the above XRD method conditions at room temperature.

### Example 1: Hot-melt extrusion (Pitolisant : Copovidone 1:3)

| **Ingredients** | **mg / unit** |
|---|---|
| **Stage-A: Hot Melt Extrusion** | |
| Pitolisant HCl | 20.00 |
| Copovidone (Kollidon VA64) | 60.00 |

| **Stage-B: Lubrication** | |
|---|---|
| Microcrystalline Cellulose | 34.12 |
| (Comprecel 102) | |
| Crospovidone type A (Polyplasdone XL) | 14.40 |
| Syloid AL1 FP | 2.40 |
| Talc (Luzenac Pharma) | 2.40 |
| Magnesium stearate | 2.40 |

| ***Core tablet weight*** | ***128.00*** |
|---|---|
| **Stage-D: Film Coating** | |
| Opadry II 85F580019 | 8.00 |
| Purified Water | q.s |
| ***Coated tablet weight*** | ***136.00*** |

Crystalline pitolisant hydrochloride and Copovidone (Kollidon VA64) of stage A were blended for 10 min in 1L blender at 20 rpm. The mixture was subjected to hot melt extrusion at 130°C. The obtained solid dispersion in form of extrudes was milled using qudra comill fitted with 32G screen at 5000 rpm. Microcrystalline cellulose, crospovidone, syloid, talc and magnesium stearate were sifted and blended with the extrudate for 10 mins at 20 rpm. The mixture was subjected to compression to obtain a tablet, which was finally film-coated.

The resulting compositions (film-coated tablets) were analyzed with respect to their impurity profile using liquid chromatography with UV detection at 220 nm. As analytical column, a stainless steel column 150 mm long, 4.6 mm internal diameter filled with Octadecylsilyl silica gel particles of 3.5 µm diameter is used.

HPLC analysis is performed in gradient mode:

| **Time (minutes)** | **% Mobile phase-A** | **% Mobile phase-B** |
|---|---|---|
| 0 | 100 | 0 |
| 30 | 100 | 0 |
| 35 | 10 | 90 |
| 45 | 10 | 90 |
| 46 | 100 | 0 |
| 60 | 100 | 0 |

Mobile phase-A: Buffer/ Solvent 53:47 (v/v)
Mobile phase-B: Buffer/ Solvent 20:80 (v/v)
Buffer: 6.0 g/L g of sodium perchlorate anhydrous in water, pH adjusted to 2.5 with perchloric acid.
Solvent: Methanol/ Acetonitrile 75:25 (v/v)
Flow rate: 1.0 mL/minute
Injection volume: 20 µL
Column oven temperature: 40 °C

The tablets contained 0.17% of total impurities. The tablet was also analysed by XRPD. It contained pitolisant HCl in amorphous form.

### Example 2: Hot-melt extrusion (Pitolisant : MCC : Copovidone 1:3:4)

| **Ingredients** | **mg / unit** |
|---|---|
| **Stage-A: Hot Melt Extrusion** | |
| Pitolisant HCl | 20.00 |
| Microcrystalline Cellulose (Comprecel 102) | 60.00 |
| Copovidone (Kollidon VA64) | 80.00 |

| **Stage-B: Lubrication** | |
|---|---|
| Microcrystalline Cellulose | 37.12 |
| (Comprecel 102) | |
| Crospovidone type A (Polyplasdone XL) | 11.2 |
| Syloid AL1 FP | 6.72 |
| Talc (Luzenac Pharma) | 4.48 |
| Magnesium stearate | 4.48 |

| ***Core tablet weight*** | ***224.00*** |
|---|---|
| **Stage-D: Film Coating** | |
| Opadry II 85F580019 | 12.00 |
| Purified Water | q.s |
| ***Coated tablet weight*** | ***236.00*** |

Crystalline pitolisant hydrochloride, copovidone and microcrystalline cellulose of stage A were blended for 10 min at 20 rpm. The mixture was subjected to hot melt extrusion at 140°C. The obtained extrudes were milled using qudra comill fitted with 1.00mm screen at 8000 rpm. Microcrystalline cellulose, crospovidone, syloid, talc and magnesium stearate were sifted and blended with the extrudate for 10 mins at 20 rpm. The mixture was subjected to compression to obtain a tablet, which was finally film-coated.

The tablet was analysed by HPLC in view of impurities: It contained 0.07% of total impurities. The tablet was also analysed by XRPD. It contained pitolisant HCl in amorphous form.

### Example 3: Hot-melt extrusion (Pitolisant : MCC : Copovidone 1:3:4)

| **Ingredients** | **mg / unit** |
|---|---|
| **Stage-A: Hot Melt Extrusion** | |
| Pitolisant HCl | 20.00 |
| Microcrystalline Cellulose (Comprecel 102) | 60.00 |
| Copovidone (Kollidon VA64) | 80.00 |

| **Stage-B: Lubrication** | |
|---|---|
| Microcrystalline Cellulose | 37.12 |
| (Comprecel 102) | |
| Crospovidone type A (Polyplasdone XL) | 23.20 |
| Syloid AL1 FP | 14.72 |
| Talc (Luzenac Pharma) | 4.48 |
| Magnesium stearate | 4.48 |

| ***Core tablet weight*** | ***244.00*** |
|---|---|
| **Stage-D: Film Coating** | |
| Opadry II 85F580019 | 12.00 |
| Purified Water | q.s |
| ***Coated tablet weight*** | ***256.00*** |

Crystalline pitolisant hydrochloride, copovidone and microcrystalline cellulose of stage A were blended for 10 min at 20 rpm. The mixture was subjected to hot melt extrusion at 140°C. The obtained extrudes were milled using qudra comill fitted with 1.00mm screen at 8000 rpm. Microcrystalline cellulose, crospovidone, syloid, talc and magnesium stearate were sifted and blended with the extrudate for 10 mins at 20 rpm. The mixture was subjected to compression to obtain a tablet, which was finally film-coated.

The tablet was analysed by HPLC in view of impurities: It contained 0.07% of total impurities. The tablet was also analysed by XRPD. It contained pitolisant HCl in amorphous form.

### Example 4: Hot-melt extrusion (Pitolisant : MCC : Copovidone 1:3:2.5)

| **Ingredients** | **mg / unit** |
|---|---|
| **Stage-A: Hot Melt Extrusion** | |
| Pitolisant HCl | 20.00 |
| Microcrystalline Cellulose (Comprecel 102) | 60.00 |
| Copovidone (Kollidon VA64) | 50.00 |

| **Stage-B: Lubrication** | |
|---|---|
| Microcrystalline Cellulose | 52.04 |
| (Comprecel 102) | |
| Crospovidone type A (Polyplasdone XL) | 20.00 |
| Syloid AL1 FP | 13.00 |
| Talc (Luzenac Pharma) | 4.48 |
| Magnesium stearate | 4.48 |

| ***Core tablet weight*** | ***224.00*** |
|---|---|
| **Stage-D: Film Coating** | |
| Opadry II 85F580019 | 12.00 |
| Purified Water | q.s |
| ***Coated tablet weight*** | ***236.00*** |

Crystalline pitolisant hydrochloride, copovidone and microcrystalline cellulose of stage A were blended for 10 min at 20 rpm. The mixture was subjected to hot melt extrusion at 130°C. The obtained extrudes were milled using a hammer mill fitted with 1.00mm screen at 10000 rpm. Microcrystalline cellulose, crospovidone, syloid, talc and magnesium stearate were sifted and blended with the extrudate for 10 mins at 20 rpm. The mixture was subjected to compression to obtain a tablet, which was finally film-coated.

The tablet was analysed by HPLC in view of impurities: It contained 0.13% of total impurities. The tablet was also analysed by XRPD. It contained pitolisant HCl in amorphous form.

### Example 5:

The tablets of Examples 1 to 4 were analysed in view of the form of pitolisant HCl present. In all the tablets of Examples 1 to 4, pitolisant HCl was in amorphous form.

The tablets of Examples 3 and 4 were further stored under stress conditions for 14 days. The results are summarised in Table 3 below:

**Table 3**

| | **Stress Condition** | **Period** | **XRD results** | |
|---|---|---|---|---|
| **Example** | | | **3** | **4** |
| 1 | 50°C/75%RH | 14 Days | Amorphous form* | Amorphous form* |
| 2 | 60°C/75%RH | 14 Days | Amorphous form* | Amorphous form* |
| 3 | 70°C/75%RH | 14 Days | Amorphous form | Amorphous form |
| 4 | 60°C/40%RH | 14 Days | Amorphous form | Amorphous form |
| 5 | 80°C/40%RH | 14 Days | Amorphous form | Amorphous form |
| 6 | 70°C/5%RH | 14 Days | Amorphous form | Amorphous form |

| | | | | |
|---|---|---|---|---|
| * Tablet became rubbery material | | | | |

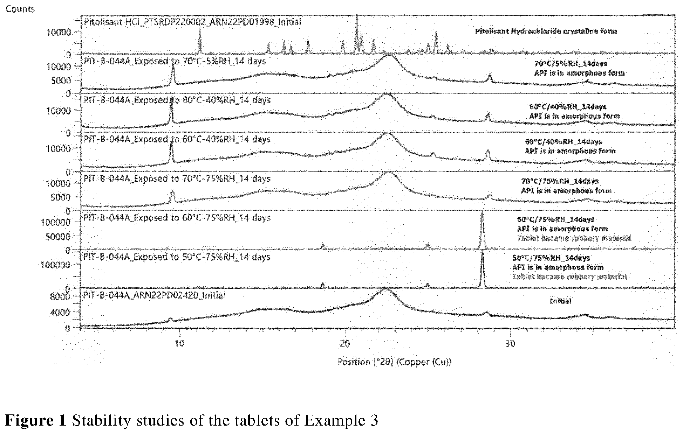

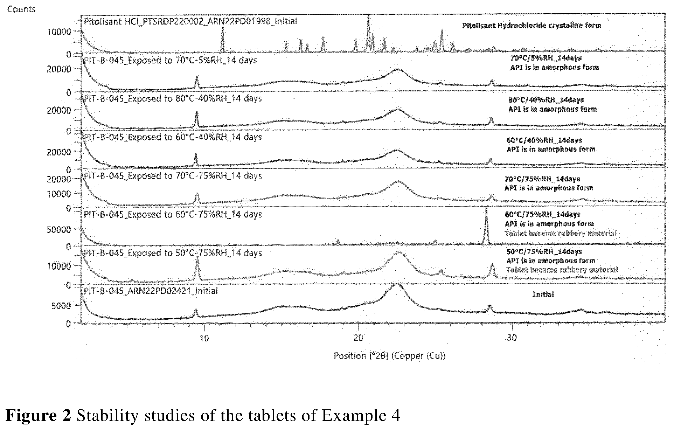

The XRPDs of the tablets before storage ("Initial") and after 14 days of storage are shown in Figures 1 and 2.

### Example 6: Spray granulation (Pitolisant : HP-β-cyclodextrin 1:3)

| **Ingredients** | **mg / unit** |
|---|---|
| **Stage-A: Granulation** | |
| Microcrystalline Cellulose (Comprecel 101) | 60.00 |
| Mannitol (Pearlitol SD 200) | 60.00 |
| Crospovidone type A (Polyplasdone XL) | 7.20 |
| Silicon dioxide hydrated (Syloid AL1 FP) | 2.40 |

| **Stage-B: Binder solution** | |
|---|---|
| Pitolisant HCl | 20.00 |
| Hydroxypropyl β-Cyclodextrin | 60.00 |
| Purified water | qs |

| **Stage-C: Lubrication** | |
|---|---|
| Crospovidone type A (Polyplasdone XL) | 7.20 |
| Silicon dioxide hydrated (Syloid AL1 FP) | 2.40 |
| Talc (Luzenac Pharma) | 2.40 |
| Magnesium stearate | 2.40 |
| ***Core tablet weight*** | ***224.00*** |

| **Stage-D: Film Coating** | |
|---|---|
| Opadry II white 85F580019 | 12.00 |
| Purified Water | |
| ***Coated tablet weight*** | ***236.00*** |

Microcristalline cellulose, Mannitol, Crospovidone and Silicondioxide of stage A were co-sifted through ASTM #20 mesh. Crystalline pitolisant hydrochloride and HP β-cyclodextrin of stage B were dissolved in water by considering 104% fluid uptake on drymix weight. Stage A material was granulated using stage B drug solution in GPCG 1.1 using the below parameters.

| **Inlet (°C)** | **Product (°C)** | **Exhaust (°C)** | **Atomization (bar)** | **Blower (%)** | **Airflow (CFM)** | **Spray (RPM)** | **RH** | |
|---|---|---|---|---|---|---|---|---|
| | | | | | | | **Inlet** | **Outlet** |
| 60 - 65 | 38 - 42 | 29 - 32 | 0.8 - 1.0 | 17 - 50 | 15 - 48 | 5 - 30 | 0 - 1 | 40-58 |

The solid dispersion in form of granules was dried at 60°C for 30 minutes after granulation and sifted through ASTM #20 mesh. The granules and stage C material was then blended for 5 min at 20 RPM. The mixture was subjected to compression to obtain a tablet, which was finally film-coated.

The granules were analysed by HPLC in view of impurities: They contained 0.07% of total impurities. The granules were also analysed by XRPD. They contained pitolisant HCl in amorphous form.

### Example 7: Spray granulation (Pitolisant : HP-β-cyclodextrin 1:2)

| **Ingredients** | **mg / unit** |
|---|---|
| **Stage-A: Granulation** | |
| Microcrystalline Cellulose (Comprecel 101) | 140.00 |
| Syloid AL1 FP | 2.40 |

| **Stage-B: Binder solution** | |
|---|---|
| Pitolisant HCl | 20.00 |
| HP Beta Cyclodextrin | 40.00 |
| Purified water | qs |

| **Stage-C: Lubrication** | |
|---|---|
| Crospovidone type A (Polyplasdone XL | 14.40 |
| Syloid AL1 FP | 2.40 |
| Talc (Luzenac Pharma) | 2.40 |
| Magnesium stearate | 2.40 |
| ***Core tablet weight*** | ***224.00*** |
| Opadry II White 85F580019 | 12.00 |
| Purified Water | |
| ***Coated tablet weight*** | ***236.00*** |

Microcrystalline cellulose and syloid of stage A were co-sifted through ASTM #20 mesh. Crystalline pitolisant hydrochloride and HP β-cyclodextrin of stage B were dissolved in water by considering 94% fluid uptake on drymix weight. Stage A material was granulated using stage B drug solution in GPCG 1.1 using the below parameters.

| **Inlet (°C)** | **Product (°C)** | **Exhaust (°C)** | **Atomization (bar)** | **Blower (%)** | **Airflow (CFM)** | **Spray (RPM)** | **RH** | |
|---|---|---|---|---|---|---|---|---|
| | | | | | | | **Inlet** | **Outlet** |
| 60 - 65 | 42 - 49 | 29 - 32 | 0.8 - 1.0 | 17 - 50 | 15 - 48 | 5 - 30 | 0 - 1 | 40-58 |

The solid dispersion in form of granules was dried at 60°C for 30 minutes after granulation and sifted through ASTM #20 mesh. The granules and stage C material was then blended for 5 min at 20 RPM. The mixture was subjected to compression to obtain a tablet, which was finally film-coated.

The granules were analysed by HPLC in view of impurities: They contained 0.07% of total impurities. The granules were also analysed by XRPD. They contained pitolisant HCl in amorphous form.

### Example 8: Spray granulation (Pitolisant : maltodextrin 1:3)

| **Ingredients** | **mg / unit** |
|---|---|
| **Stage-A: Granulation** | |
| Microcrystalline Cellulose (Comprecel 101) | 86.00 |
| Crospovidone type A (Polyplasdone XL) | 7.20 |
| Syloid AL1 FP | 4.80 |

| **Stage-B: Binder solution** | |
|---|---|
| Pitolisant HCl | 20.00 |
| Maltodextrin (Glucidex 12D) | 60.00 |
| Syloid AL1 FP | 30.00 |
| Purified water | qs |

| **Stage-C: Lubrication** | |
|---|---|
| Crospovidone type A (Polyplasdone XL) | 4.00 |
| Syloid AL1 FP | 7.52 |
| Talc (Luzenac Pharma) | 2.24 |
| Magnesium stearate | 2.24 |
| ***Core tablet weight*** | ***224.00*** |

| **Stage-D: Film Coating** | |
|---|---|
| Opadry II white 85F580019 | 12.00 |
| Purified Water | q.s |
| ***Coated tablet weight*** | ***236.00*** |

Microcrystalline cellulose, crospovidone and syloid of stage A were co-sifted through ASTM #20 mesh. Crystalline pitolisant hydrochloride and maltodextrin of stage B were dissolved in water by considering 94% fluid uptake on drymix weight and silicon dioxide was dispersed in the same solution. Stage A material was granulated using stage B drug solution in GPCG 1.1 using the below parameters.

| **Inlet (°C)** | **Product (°C)** | **Exhaust (°C)** | **Atomization (bar)** | **Blower (%)** | **Airflow (CFM)** | **Spray (RPM)** | **RH** | |
|---|---|---|---|---|---|---|---|---|
| | | | | | | | **Inlet** | **Outlet** |
| 60 - 65 | 42 - 49 | 29 - 32 | 0.8 - 1.0 | 17 - 50 | 15 - 48 | 5 - 30 | 0 - 1 | 40-58 |

The solid dispersion in form of granules was dried at 60°C for 30 minutes after granulation and sifted through ASTM #20 mesh. The granules and stage C material was then blended for 5 min at 20 RPM. The mixture was subjected to compression to obtain a tablet, which was finally film-coated.

The granules were analysed by HPLC in view of impurities: They contained 0.07% of total impurities. The granules were also analysed by XRPD. They contained pitolisant HCl in amorphous form.

### Example 9

The tablets of Examples 6-8 were analysed in view of the form of pitolisant HCl present. In all the tablets of Examples 6-8, pitolisant HCl was in amorphous form.

The tablets of Examples 6, 7 and 8 were further stored under stress conditions for 14 days. The results are summarised in Table 4 below:

**Table 4**

| | **Stress Condition** | **Period** | **XRD results** | | |
|---|---|---|---|---|---|
| **Example** | | | **6** | **7** | **8** |
| 1 | 50°C/75%RH | 14 Days | Amorphous form* | Amorphous form* | Amorphous form* |
| 2 | 60°C/75%RH | 14 Days | Amorphous form | Amorphous form | Amorphous form |
| 3 | 70°C/75%RH | 14 Days | Amorphous form | Amorphous form | Amorphous form¹⁾ |
| 4 | 60°C/40%RH | 14 Days | Amorphous form | Amorphous form | Amorphous form |
| 5 | 80°C/40%RH | 14 Days | Amorphous form | Amorphous form | Amorphous form |
| 6 | 70°C/5%RH | 14 Days | -- | Amorphous form | Amorphous form |

| | | | | | |
|---|---|---|---|---|---|
| *Tablet became rubbery material ¹⁾ storage was carried out for 2 days | | | | | |

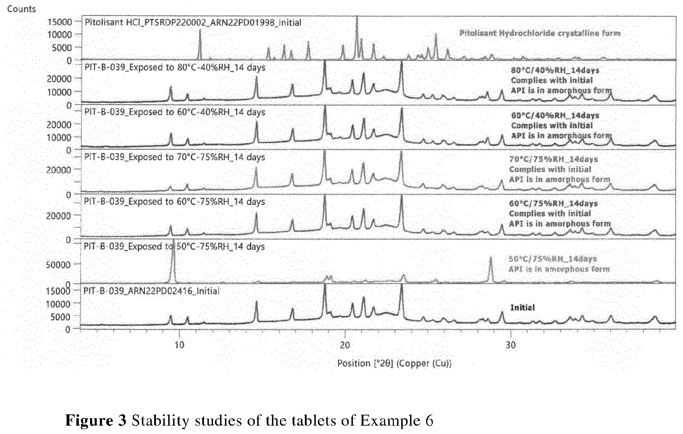

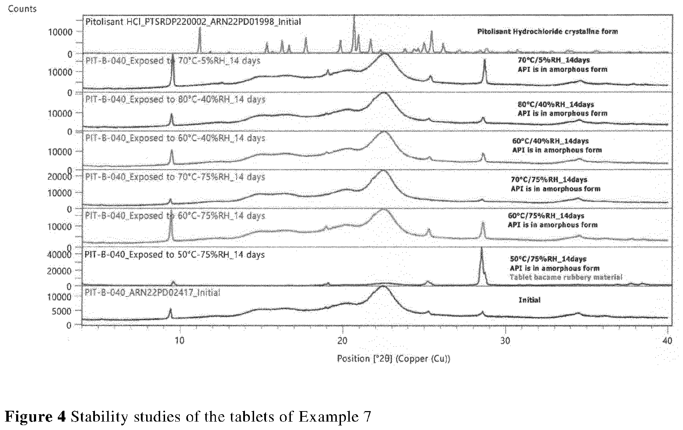

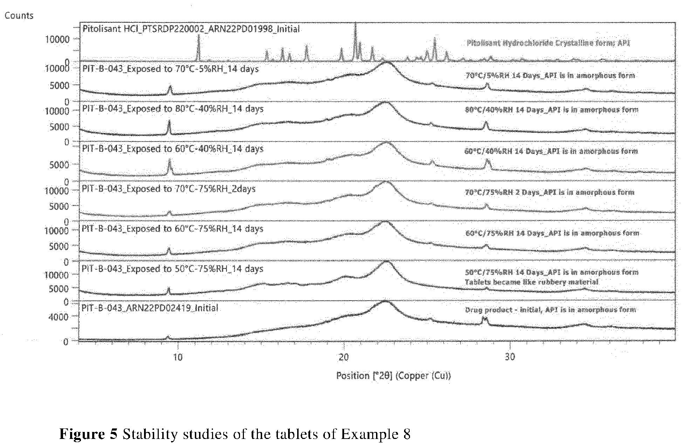

The XRPDs of the tablets before storage ("Initial") and after 14 days of storage are shown in Figures 3-5.

### Example 10: Stability data after storage for 1 month and 6 months under stressed conditions

The tablets of Examples 1, 3 and 4 as well as 6 to 8 were stored for 1 month and 6 months, respectively, under stressed conditions (40°C, 75% RH), and were packed in different materials.

The data is summarized in table 5 below.

**Table 5**

| **Ex.** | **Pack** | **XRD result** | | | **Total Impurities** | |
|---|---|---|---|---|---|---|
| | | **Initial** | **40°C/75% RH, 1M** | **40°C/75% RH, 6M** | **Initial** | **40°C/75%RH, 1M** |
| 6 | Alu-Alu-Blister | Amorphous | Amorphous form | Amorphous form | 0.07 | 0.06 |
| | HDPE (2 g) | | | | | 0.06 |
| | HDPE (3 g) | | | | | 0.06 |
| 7 | HDPE (2 g) | Amorphous form | Amorphous form | Amorphous form | 0.07 | 0.06 |
| 8 | Alu-Alu-Blister | Amorphous form | Amorphous form | Amorphous form | 0.07 | 0.06 |
| | HDPE (2 g) | | | | | 0.06 |
| 1 | HDPE (2 g) | Amorphous form | Amorphous form | Amorphous form | n.d. | n.d. |
| | HDPE (3 g) | Amorphous form | Amorphous form | Amorphous form | | |
| 3 | Alu-Alu-Blister | Amorphous form | Amorphous form | Amorphous form | n.d. | n.d. |
| 4 | Alu-Blister | Amorphous form | Amorphous form | - | 0.13 | 0.18 |
| | HDPE (2 g) | | | Amorphous form | | 0.51 |

The results show that the amorphous form was stabilized in the tablets according to the present invention. Even after 6 months at 40°/75% RH, the amorphous form did not reconvert to a crystalline form.

In addition, while the impurity profile for all the tablets was acceptable, tablets containing the solid dispersion prepared by spray granulation had lower levels of impurities compared to tablets containing the solid dispersion prepared by hot-melt extrusion.

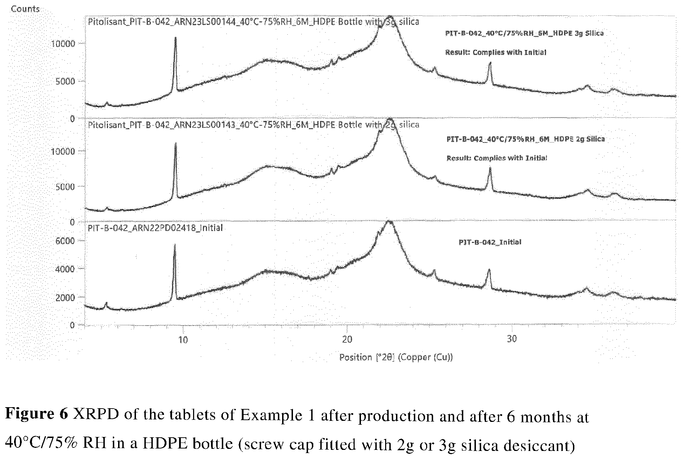

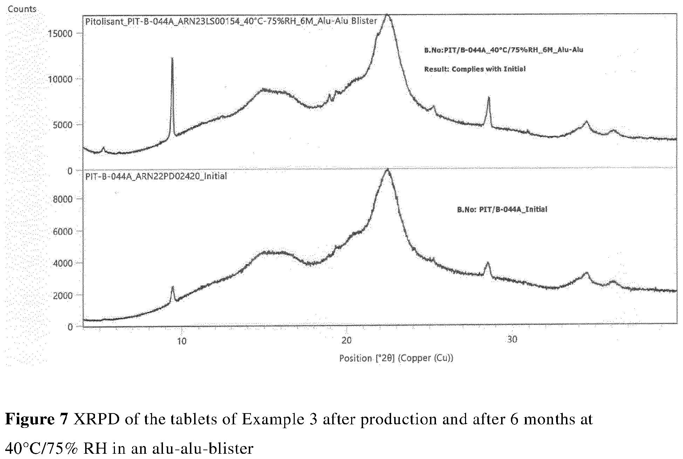

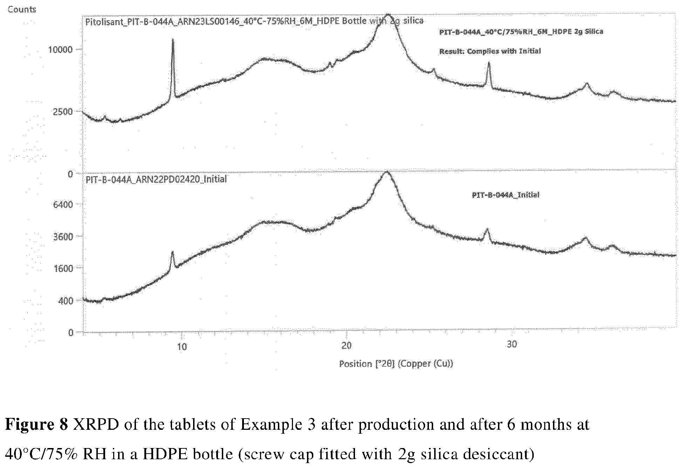

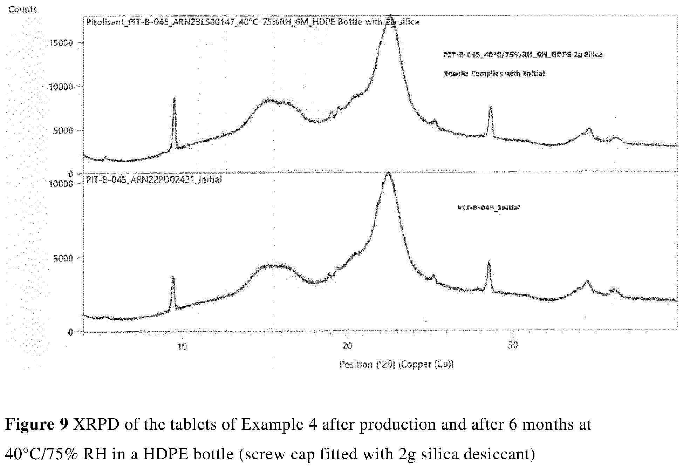

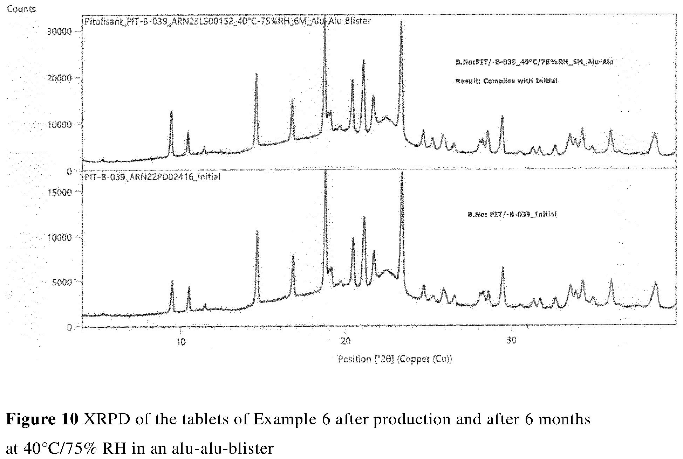

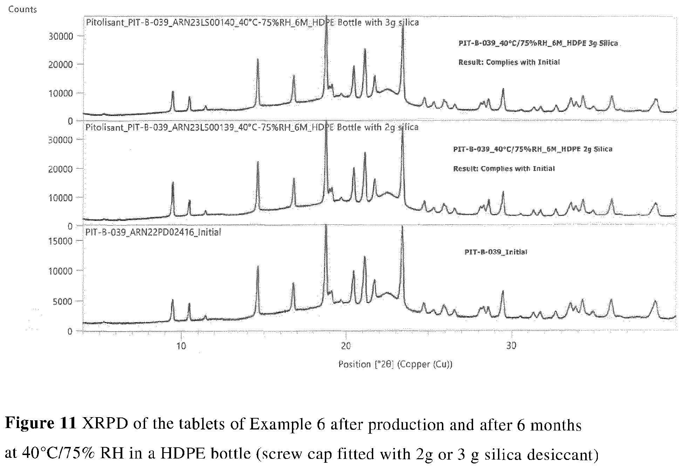

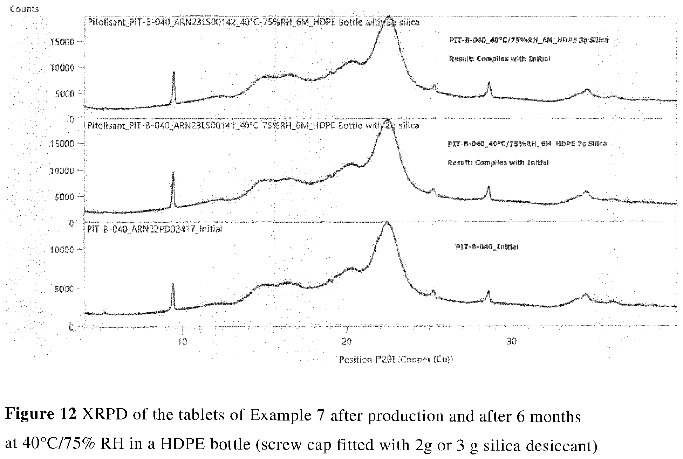

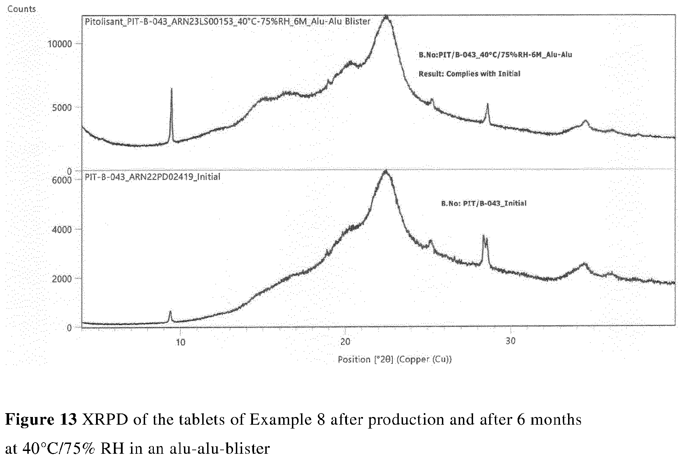

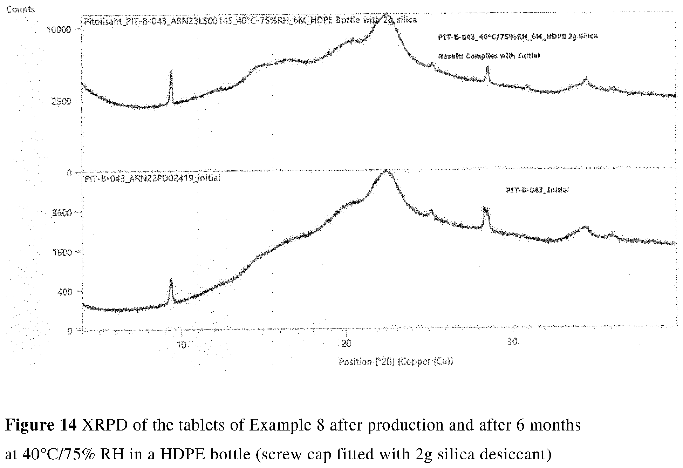

The XRPDs of the tablets before storage ("Initial") and after 6 months of storage are shown in Figures 6-14.

### Example 11: Premix of pitolisant HCl and polyvinylpyrrolidone (1:4)

In an uncapped vial, one part of crystalline pitolisant HCl was mixed with four parts polyvinylpyrrolidone (Kollidon^{®} 25), and dissolved at 25°C in a sufficient amount of a 1 : 1 mixture of water and ethanol, until all powders are in solution.

Using a rotary vacuum evaporator the solvent was removed at 70°C. The dry residue was removed from the evaporation flask and sieved (630 µm), to obtain a solid premix of amorphous pitolisant HCl and polyvinylpyrrolidone.

Analysis by XRPD confirmed that pitolisant HCl was present in amorphous form.

### Example 12: Premix of pitolisant HCl and polyvinylpyrrolidone (1:7)

One part of crystalline pitolisant HCl and seven parts of polyvinylpyrrolidone (Kollidon^{®} 25) were dissolved at 25°C in 30 parts of a 80:20 acetone/ethanol mixture (w/w). Using a rotary vacuum evaporator the solvent was removed at 70°C. The dry residue was removed from the evaporation flask and sieved (630 µm), to obtain a solid premix of amorphous pitolisant HCl and polyvinylpyrrolidone.

Analysis by XRPD confirmed that pitolisant HCl was present in amorphous form.

### Example 13: Premix of pitolisant HCl and β-cyclodextrine (1:3)

One part of crystalline pitolisant HCl and three parts of β-cyclodextrine were dissolved at 25°C in 30 parts of a 1:1 acetone/ethanol mixture (w/w). Using a rotary vacuum evaporator the solvent was removed at 70°C.

The dry residue was removed from the evaporation flask and sieved (630 µm), to obtain a solid premix of amorphous pitolisant HCl and β-cyclodextrine.

Analysis by XRPD confirmed that pitolisant HCl was present in amorphous form.

### Example 14: Premix of pitolisant HCl and mannitol (1:3)

1 g of crystalline pitolisant HCl and 3 g of mannitol (Pearlitol SD 200) were weighed. This mixture was dissolved at 25°C in 100 ml of ethanol and then evaporated in a rotary vacuum evaporator. The solid product was screened through a 0.5 mm sieve and subsequently dried in a vacuum drying oven at 10 kPa for 24 hours.

Analysis by XRPD confirmed that pitolisant HCl was present in amorphous form.

### Example 15: Premix of pitolisant HCl and mannitol (5:1)

0.5 g of crystalline pitolisant HCl and 0.1 g of mannitol were dissolved at 25°C in 100 mL water. The solution was then concentrated in rotavapor apparatus under vacuum at 45 to 55°C to get a solid. Then dried the obtained solid in rotavapor apparatus under vacuum at 60°C to get pitolisant HCl premix with mannitol.

Analysis by XRPD confirmed that pitolisant HCl was present in amorphous form.

### Example 16: Tablet comprising solid premix solid premix of Example 14

| **Ingredients** | **mg / unit** | |
|---|---|---|
| **Stage-A: Premix** | | |
| Pitolisant HCl | 20.00 | |
| Mannitol (Pearlitol SD 200) | 60.00 | |

| **Stage-B: Lubrication** | | |
|---|---|---|
| Microcrystalline Cellulose (Comprecel 102) | 34.12 | |
| Crospovidone type A (Polyplasdone XL) | 14.40 | |
| Syloid AL1 FP | 2.40 | |
| Talc (Luzenac Pharma) | 2.40 | |
| Magnesium stearate | 2.40 | |
| ***Core tablet weight*** | ***128.00*** | |

| **Stage-D: Film Coating** | | |
|---|---|---|
| Opadry II 85F580019 | 8.00 | |
| Purified Water | q.s | |
| ***Coated tablet weight*** | ***136.00*** | |

Microcrystalline cellulose, crospovidone, syloid, talc and magnesium stearate were sifted and blended with the solid premix of Example 14 for 10 mins at 20 rpm. The mixture was subjected to compression to obtain a tablet, which was finally film-coated.

The tablet was analysed by XRPD. It still contained pitolisant HCl in amorphous form.

### Comparative Example 17: Tablet containing crystalline pitolisant HCl

| **Ingredients** | |
|---|---|
| **Stage-A (Blending)** | **Mg/tablet** |
| Crystalline pitolisant HCl | 5.00 |
| Cellulose, Microcrystalline (*Comprecel M102*) | 23.25 |
| Crospovidone *(Polyplasdone XL)* | 2.19 |
| Colloidal silicon dioxide *(Aerosil 200 Pharma)* | 0.31 |
| Talc (Luzenac Pharma) | 0.63 |
| Magnesium Stearate | 0.63 |
| **Core Tablet Weight (mg)** | **32.00** |

| **Stage-B (Coating)** | |
|---|---|
| Opadry II 85F580019 | 2.00 |
| Purified, water | q.s. |
| **Coated Tablet Weight (mg)** | **34.00** |

The crystalline form of pitolisant HCl used corresponded to the crystalline form disclosed in WO2006084833A1.

Crystalline pitolisant HCl and all the ingredients of stage A, except for magnesium stearate, were milled using a suitable screen (e.g. 24R) and blended to get uniform mixture. This blend was then blended with magnesium stearate to obtain the lubricated blend, and compressed into tablets which were finally coated.

Dissolution tests (0.1N HCl, Paddle, 1000 ml at 75 rpm) resulted in a 67 % release of pitolisant after 5 min and of 100 % after 10 min.

### Comparative Example 18: Tablet containing crystalline pitolisant HCl

| **Ingredients** | |
|---|---|
| **Stage-A (Blending)** | **Mg/tablet** |
| Crystalline pitolisant HCl | 20.00 |
| Colloidal silicon dioxide *(Aerosil 200 Pharma)* | 93.00 |
| Cellulose, Microcrystalline *(Comprecel M102)* | 8.75 |
| Crospovidone *(Polyplasdone XL)* | 1.25 |
| Talc (Luzenac Pharma) | 2.50 |
| Magnesium Stearate | 2.50 |
| **Core Tablet Weight (mg)** | **128.00** |

| **Stage-B (Coating)** | |
|---|---|
| Opadry II 85F580019 | 8.00 |
| Purified, water | q.s. |
| **Coated Tablet Weight (mg)** | **136.00** |

The crystalline form of pitolisant HCl used corresponded to the crystalline form disclosed in WO2006084833A1.

Crystalline pitolisant HCl and all the ingredients of stage A, except for magnesium stearate, were milled using a suitable screen (e.g. 24R) and blended to get uniform mixture. This blend was then blended with magnesium stearate to obtain the lubricated blend, and compressed into tablets which were finally coated.

Dissolution tests (0.1N HCl, Paddle, 1000 ml at 75 rpm) resulted in a 97 % release of pitolisant after 5 min and of 100 % after 10 min.

### Example 19: Premix of pitolisant HCl and syloid (1:1)

1.0 g of crystalline pitolisant HCl were dissolved at 25°C in 100 mL ethanol. 1.0 g mesoporous silicon dioxide (syloid) were added to the solution. The solvent was evaporated under vacuum at 65 °C and dried under vacuum for about 18 hours at 40 °C to afford pitolisant HCl premix with syloid.

### Example 20: Premix of pitolisant HCl and magnesium aluminometasilicate (2:1)

0.5 g of crystalline pitolisant HCl were dissolved at 25°C in 100 mL water. 0.25 g magnesium aluminometasilicate (Neusilin^{®}) were added to the solution. The solvent was evaporated under vacuum at 65 °C and dried under vacuum for about 18 hours at 40 °C to afford pitolisant HCl premix with aluminometasilicate.

## Claims

1. Pharmaceutical mixture comprising
a) pitolisant free base or a pharmaceutically acceptable salt thereof, wherein pitolisant free base or a pharmaceutically acceptable salt thereof is present in the mixture in amorphous form, and
b) at least one pharmaceutically acceptable excipient.

2. Pharmaceutical mixture form according to claim 1, wherein the at least one pharmaceutically acceptable excipient comprises a diluent, a binder, a plasticizer or a disintegrant.

3. Pharmaceutical mixture according to claim 1 or 2,
wherein the at least one pharmaceutically acceptable excipient is selected from the group comprising gum arabic, sodium alginate, propylene glycol alginate, agar, gelatin, tragacanth, xanthan gum, polyvinylpyrrolidone, poly(vinylpyrrolidone/vinylacetate), polyvinylcaprolactam/polyvinylacetate/polyethylene glycol graft copolymer, polyethylene glycol/polyvinyl alcohol graft copolymer, polyvinyl alcohol, polyethylene oxide, polyethylene oxide/propylene oxide), macrogolglycerol hydroxystearate, D-atocopheryl polyethylene glycol succinate, poly(butyl methacrylate/2-dimethylaminoethyl methacrylate/methyl methacrylate), poly(ethyl acrylate/methyl methacrylate), poly(ethyl acrylate/methyl methacrylate/trimethylammonioethyl methacrylate chloride), polyethylene glycol, methyl cellulose, hydroxypropyl methylcellulose, hydroxypropyl cellulose, hydroxyethyl cellulose, carboxymethyl ethyl cellulose, sodium carboxymethyl cellulose, starch, pregelatinized starch, maltodextrins, cyclodextrins, sodium starch glycolate, colloidal silicon dioxide, carboxymethylcellulose, polyvinyl acetate, gelatins, hypromellose phthalate, polyols and mixtures thereof.

4. Pharmaceutical mixture according to anyone of claims 1 to 3,
wherein the at least one pharmaceutically acceptable excipient comprises mesoporous inorganic hygroscopic-stability increasing substances.

5. Pharmaceutical mixture according to claim 4,
wherein the mesoporous inorganic hygroscopic-stability increasing substances are selected from silica, magnesium aluminometasilicate and magnesium carbonate.

6. Pharmaceutical mixture according to anyone of claims 1 to 5,
wherein the pharmaceutical mixture is a solid dispersion, wherein pitolisant free base or a pharmaceutically acceptable salt thereof is dispersed in a matrix containing the at least one pharmaceutically acceptable excipient, and wherein the solid dispersion is prepared by hot-melt extrusion.

7. Pharmaceutical mixture according to claim 6,
wherein the hot-melt extrusion is carried out at a temperature from 120 to 170°C.

8. Pharmaceutical mixture according to claims 6 or 7,
wherein the weight ratio of pitolisant free base or a pharmaceutically acceptable salt thereof to the at least one pharmaceutically acceptable excipient is 10:1 to 1:10.

9. Pharmaceutical mixture according to anyone of claims 1 to 5,
wherein the pharmaceutical mixture is a solid dispersion, wherein pitolisant free base or a pharmaceutically acceptable salt thereof is dispersed in a matrix containing the at least one pharmaceutically acceptable excipient, and wherein the solid dispersion is prepared by spray granulation.

10. Pharmaceutical mixture according to claims 9,
wherein the weight ratio of pitolisant free base or a pharmaceutically acceptable salt thereof to the at least one pharmaceutically acceptable excipient is 10:1 to 1:10.

11. Pharmaceutical mixture according to anyone of claims 1 to 5,
wherein the pharmaceutical mixture is a solid premix.

12. Oral dosage form comprising the pharmaceutical mixture according to any one of claims 1 to 11 and optionally further pharmaceutical excipient(s).

13. Oral dosage form according to claim 12, wherein the content release of the dosage form after 15 minutes is 70 % to 100 % determined by USP method (apparatus 2; paddle, 1000 ml of 0.1 N HCl and 37°C, 75 rpm).

14. Oral dosage form according to claims 12 or 13, wherein the oral dosage form contains 4.45 mg or 17.8 mg of pitolisant based on the free base.

15. Oral dosage form according to any one of claims 12 to 14, wherein the oral dosage form is a tablet.
